# EUROPEAN PATENT SPECIFICATION

(11) **EP 2 916 809 B1**
(45) Date of publication and mention of the grant of the patent: **25.12.2019**
(21) Application number: 13853507.5
(22) Date of filing: 08.11.2013
(51) Int. Cl.: A61K 8/36, A61K 8/365, A61K 8/58, A61Q 5/04, A61K 8/73, A61K 8/81, A61Q 5/06, A61K 8/44

(54) **COMPOSITION COMPRISING A DICARBONYL COMPOUND AND PROCESS FOR STRAIGHTENING THE HAIR USING THIS COMPOSITION**
ZUSAMMENSETZUNG MIT EINER DICARBONYLVERBINDUNG UND HAARGLÄTTUNGSVERFAHREN UNTER VERWENDUNG DIESER ZUSAMMENSETZUNG
COMPOSITION COMPRENANT UN COMPOSÉ DICARBONYLE ET PROCÉDÉ DE DÉFRISAGE DES CHEVEUX AU MOYEN DE CETTE COMPOSITION

(30) Priority: 09.11.2012 FR 1260673
(43) Date of publication of application: 16.09.2015
(73) Proprietor: L'OREAL, 75008 Paris (FR)
(72) Inventor: BIATO, Camila, CEP-22795-295 Rio de Janeiro RJ (BR); SILVESTRE, Liliane, CEP-21235-602 Rio de Janeiro RJ (BR)
(74) Representative: L'Oreal
(86) International application number: PCT/EP2013/073419
(87) International publication number: WO 2014/072479

(56) References cited:
- WO-A1-91/04007
- WO-A1-2007/135299
- WO-A1-2012/105985
- WO-A2-2011/104282
- WO-A2-2012/010351
- FR-A1- 2 585 947
- GB-A- 2 114 616
- DATABASE GNPD [Online] MINTEL; January 2012 (2012-01), "Moroccan Relaxing Treatment with Argan Oil", XP002703623, Database accession no. 1692744

## Description

The present invention relates to a cosmetic composition, especially a hair composition, based on one or more particular dicarbonyl compounds, and also to a process for straightening keratin fibres, especially the hair, using this composition.

In the hair field, consumers wish to have available compositions which make it possible to introduce a temporary change to their head of hair, while targeting good remanence of the effect produced. In general, it is desirable for the change to withstand shampooing operations for a minimum of 15 days, indeed even more, depending on the nature of the said change.

Treatments already exist for modifying the colour or the shape of the hair and also, to some extent, the texture of the hair. One of the known treatments for modifying the texture of the hair consists of the combination of heat and of a formaldehyde-comprising composition. This treatment is especially effective for imparting a better appearance to damaged hair and/or for treating long hair and curly hair.

The action of formaldehyde is associated with its ability to crosslink proteins by reaction with the nucleophilic sites thereof. The heat used may be that of an iron (flat tongs or crimping iron), the temperature of which may generally be up to 200°C or more. However, it is increasingly sought to avoid the use of such substances, which may prove to be aggressive to the hair and other keratin materials.

Patent application WO 2011/104 282 thus proposed a novel process for semi-permanently straightening the hair, which consists in applying an α-keto acid solution to the hair for 15 to 120 minutes, then drying and, finally, straightening the head of hair with an iron at a temperature of about 200°C. The α-keto acid employed is preferably glyoxylic acid.

Patent application WO2012/105985 discloses a method of enhancing the removal of the natural curl from hair comprising the steps of applying an alkaline composition and then an acidic composition having a pH of less than 1,5 which comprises glyoxylic acid and/or glyoxylic amide.

However, it has been noted that the use of glyoxylic acid can result in some significant limitations; in particular, at high concentration it may not be well tolerated, in particular when the scalp is sensitive and/or irritated. Its volatility, amplified by the use of heat (iron), can also be a problem. Furthermore, cosmetic formulations having an acidic pH may impair the hair and/or impair its colour.

It is already known practice to use glyoxylic acid esters in hair compositions, in particular in hair dye compositions, as described in document DE19859722, and in reducing compositions, as described in document DE 19860239. Patent application WO 2007/135299 discloses methods for straightening the hair comprising the application on hair of a composition based on alpha-hydroxy and/or keto acid derivative. WO 2012/010351 discloses methods for straightening the hair comprising the application on hair of a composition based on glyoxylic acid.

However, the efficacy of these compositions is not yet sufficient.

The aim of the invention is to develop a straightening/relaxing composition which is stable over time and which makes it possible to straighten/relax and/or reduce the volume of the hair in an efficient and persistent manner while limiting damage to the hair, while at the same time retaining comfort at the time of application for the user of the composition, but also for the hairdresser who applies it.

Thus, one subject of the present invention is a cosmetic composition comprising at least one cationic polymer or at least one cationic polymer and one amphoteric polymer and at least 3% by weight relative to the total weight of the composition of one or more dicarbonyl compounds corresponding to formula (I) below and/or hydrates thereof and/or salts thereof: 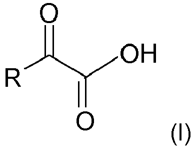 in which formula (I):
R represents an atom or group chosen from i) hydrogen, ii) carboxyl -C(O) OH, iii) linear or branched C₁-C₆ alkyl which is optionally substituted, preferably with at least one hydroxyl -OH radical, carboxyl -C(O)-OH radical or halogen radical such as Br; iv) optionally substituted phenyl, v) optionally substituted benzyl, iv) and v) preferably being optionally substituted with at least one -OH or -C(O) OH radical; vi) an indolyl radical and vii) an imidazolylmethyl radical and tautomers thereof such as 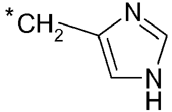 with * representing the part linked to the rest of the molecule.

A subject of the invention is also a process for straightening keratin fibres, especially the hair, which comprises the application to the hair of the composition of the invention, followed by a straightening step using a straightening iron at a temperature of at least 150°C, preferably ranging from 150 to 250°C.

The composition of the invention is stable. The composition of the invention and the process for treating keratin fibres using it allow good straightening of keratin fibres while limiting damage to these keratin fibres, even when the application of the composition is followed by a heat treatment, in particular using a hair-straightening iron, and have an appreciated wear quality, in particular without excessive vaporization of the composition at the time of straightening. The composition and the process for treating keratin fibres according to the invention also make it possible to limit the change in the colour of the fibres and also the problems of breaking of the fibres such as the hair. The composition and the process of the invention will also improve the physical properties of the hair, by reducing the frizziness effect in a long-lasting manner.

In the text hereinbelow, the term "at least one" is equivalent to the term "one or more".

Preferably, the composition according to the invention comprises neither a colouring agent nor a reducing agent.

According to the present invention, the term "colouring agents" means agents for colouring keratin fibres such as direct dyes, pigments or oxidation dye precursors (bases and couplers). If they are present, their content does not exceed 0.001% by weight relative to the total weight of the composition. Indeed, at such a content, only the composition would be dyed, i.e. no dyeing effect would be observed on the keratin fibres.

It is recalled that oxidation dye precursors, oxidation bases and couplers are colourless or sparingly coloured compounds, which, via a condensation reaction in the presence of an oxidizing agent, give a coloured species. With regard to direct dyes, these compounds are coloured and have a certain affinity for keratin fibres.

According to the present invention, the term "reducing agent" means an agent that is capable of reducing the disulfide bonds of the hair, such as compounds chosen from thiols, alkali metal sulfites, hydrides and phosphines.

In the present invention, the dicarbonyl compounds of formula (I) may be in free form, but also in their hydrate forms or in the form of their salts, preferably in free form or in the form of hydrates.

The salts may be salts derived from the interaction of the compounds of formula (I) with acids or bases, it being possible for the acids or bases to be of organic or mineral nature.

Preferably, the salts are salts derived from the interaction of the compounds of formula (I) with bases. Mention will be made in particular of the salts of alkali metals or alkaline-earth metals and in particular the sodium salts.

Preferably, the dicarbonyl compound(s) corresponding to formula (I) and/or hydrates thereof and/or salts thereof are chosen from the dicarbonyl compounds corresponding to formula (I) in which R represents i) a hydrogen atom or ii) a linear or branched C₁-C₆ alkyl group optionally substituted with a carboxyl group.

More preferably, they are chosen from glyoxylic acid and pyruvic acid and hydrates thereof or salts thereof and more preferentially from glyoxylic acid, and the hydrate forms of this compound.

Mention may be made of glyoxylic acid and also the hydrate form thereof (HO)₂CH-C(O)-OH, for instance the glyoxylic acid as a 50% aqueous solution sold by the company Merck.

According to a particularly preferred embodiment, the compound of formula (I) is glyoxylic acid in hydrate form.

According to one embodiment, the composition of the invention comprises from 3% to 15% of one or more dicarbonyl compounds corresponding to formula (I) and/or of hydrate forms thereof and/or salts thereof, preferably from 5% to 15% and preferentially from 5% to 10% by weight relative to the total weight of the composition.

According to the invention, the composition comprises at least one cationic polymer or at least one cationic polymer and one amphoteric polymer.

### Cationic polymer

It is recalled that, for the purposes of the present invention, the term "cationic polymer" denotes any polymer containing cationic groups and/or groups that can be ionized into cationic groups.

Preferably, the cationic polymer present in the composition according to the invention is a linear, random, graft or block homopolymer or copolymer and comprises at least one cationic group and/or group that can be ionized into a cationic group chosen from primary, secondary, tertiary and/or quaternary amine groups that form part of the main polymer chain or that are borne by a side substituent directly connected thereto.

Preferably, the cationic charge density of the cationic polymers according to the invention is greater than 1 meq./g and even more particularly greater than or equal to 4 meq./g. This charge density is determined by the Kjeldahl method. It may also be calculated from the chemical nature of the polymer.

The cationic polymers used generally have a number-average molecular weight of between 500 and 5 x 10⁶ approximately and preferably between 10³ and 3 x 10⁶.

Among the cationic polymers, mention may more particularly be made of polymers of the polyamine, polyaminoamide and polyquaternary ammonium type.

These are known products and are especially described in patents FR 2505348 or FR 2542997.

Among the cationic polymers that may be used in the context of the invention, mention may be made of the following polymers, alone or as a mixture:
(1) Homopolymers or copolymers derived from acrylic or methacrylic esters or amides and comprising at least one of the units of formula (I), (II), (III) or (IV) below: 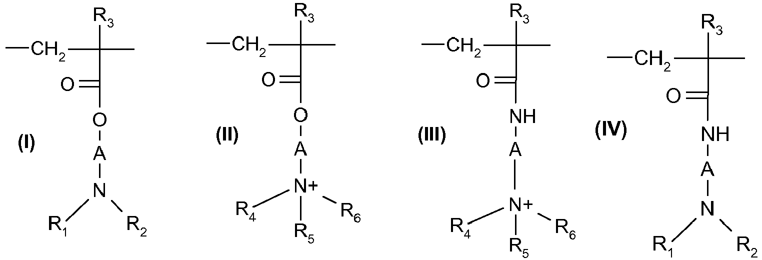 in which:
   R₃, which may be identical or different, denotes a hydrogen atom or a CH₃ radical;
   A, which may be identical or different, represents a linear or branched C₁-C₆ and preferably C₂-C₃ alkyl group or a C₁-C₄ hydroxyalkyl group;
   R₄, R₅ and R₆, which may be identical or different, represent a C₁-C₁₈ alkyl group or a benzyl radical, and preferably a C₁-C₆ alkyl group;
   R₁ and R₂, which may be identical or different, represent hydrogen or a C₁-C₆ alkyl group, and preferably methyl or ethyl;
   X denotes an anion derived from a mineral or organic acid, such as a methosulfate anion or a halide such as chloride or bromide.

   The polymers of family (1) may also contain one or more units derived from comonomers which may be chosen from the family of acrylamides, methacrylamides, diacetone acrylamides, acrylamides and methacrylamides substituted on the nitrogen with lower (C₁-C₄) alkyls, acrylic or methacrylic acids or esters thereof, vinyllactams such as vinylpyrrolidone or vinylcaprolactam, and vinyl esters. Thus, among these polymers of family (1), mention may be made of:
   - copolymers of acrylamide and of dimethylaminoethyl methacrylate quaternized with dimethyl sulfate or with a dimethyl halide, such as the product sold under the name Hercofloc by the company Hercules,
   - the copolymers of acrylamide and of methacryloyloxyethyltrimethylammonium chloride described, for example, in EP 80 976 and sold under the name Bina Quat P 100 by the company Ciba Geigy,
   - the copolymer of acrylamide and of methacryloyloxyethyltrimethylammonium methosulfate sold under the name Reten by the company Hercules,
   - quaternized or non-quaternized vinylpyrrolidone/dialkylaminoalkyl acrylate or methacrylate copolymers, such as the products sold under the name Gafquat by the company ISP, for instance Gafquat 734 or Gafquat 755, or alternatively the products known as Copolymer 845, 958 and 937. These polymers are described in FR 2 077 143 and FR 2 393 573,
   - dimethylaminoethyl methacrylate/vinylcaprolactam/vinylpyrrolidone terpolymers, such as the product sold under the name Gaffix VC 713 by the company ISP,
   - vinylpyrrolidone/methacrylamidopropyldimethylamine copolymers sold in particular under the name Styleze CC 10 by ISP,
   - quaternized vinylpyrrolidone/dimethylaminopropylmethacrylamide copolymers such as the product sold under the name Gafquat HS 100 by the company ISP, and
   - the crosslinked polymers of methacryloyloxy(C₁-C₄)alkyl tri(C₁-C₄)alkylammonium salts, such as the polymers obtained by homopolymerization of dimethylaminoethyl methacrylate quaternized with methyl chloride, or by copolymerization of acrylamide with dimethylaminoethyl methacrylate quaternized with methyl chloride, the homo- or copolymerization being followed by crosslinking with an olefinically unsaturated compound, in particular methylenebisacrylamide. Use may be made more particularly of a crosslinked acrylamide/methacryloyloxyethyltrimethylammonium chloride copolymer (20/80 by weight) in the form of a dispersion containing 50% by weight of the said copolymer in mineral oil. This dispersion is sold under the name Salcare® SC 92 by the company Ciba. Use may also be made of a crosslinked homopolymer of methacryloyloxyethyltrimethylammonium chloride containing approximately 50% by weight of the homopolymer in mineral oil or in a liquid ester. These dispersions are sold under the names Salcare® SC 95 and Salcare® SC 96 by the company Ciba.
(2) Cationic cellulose derivatives such as cellulose copolymers or cellulose derivatives grafted with a water-soluble quaternary ammonium monomer, and described in particular in US 4 131 576, such as hydroxyalkyl celluloses, for instance hydroxymethyl, hydroxyethyl or hydroxypropyl celluloses grafted in particular with a methacryloylethyltrimethylammonium, methacrylamidopropyltrimethylammonium or dimethyldiallylammonium salt.
   The commercial products corresponding to this definition are more particularly the products sold under the names Celquat L 200 and Celquat H 100 by the company National Starch.
(3) Cationic guar gums described more particularly in US 3 589 578 and US 4 031 307, such as guar gums containing cationic trialkylammonium groups. Use is made, for example, of guar gums modified with a 2,3-epoxypropyltrimethylammonium salt (for example, chloride).
   Such products are sold especially under the trade names Jaguar C13S, Jaguar C15, Jaguar C17 and Jaguar C162 by the company Meyhall.
(4) Polymers consisting of piperazinyl units and of divalent alkylene or hydroxyalkylene radicals containing straight or branched chains, optionally interrupted with oxygen, sulfur or nitrogen atoms or with aromatic or heterocyclic rings, and also the oxidation and/or quaternization products of these polymers. Such polymers are described, in particular, in FR 2 162 025 and FR 2 280 361.
(5) Water-soluble polyaminoamides prepared in particular by polycondensation of an acidic compound with a polyamine; these polyaminoamides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent being used in proportions ranging from 0.025 to 0.35 mol per amine group of the polyaminoamide; these polyaminoamides may be alkylated or, if they contain one or more tertiary amine functions, they may be quaternized. Such polymers are described, in particular, in FR 2 252 840 and FR 2 368 508.
   Polyaminoamide derivatives resulting from the condensation of polyalkylene polyamines with polycarboxylic acids followed by alkylation with difunctional agents. Mention may be made, for example, of adipic acid/dialkylaminohydroxyalkyldialkylenetriamine polymers in which the alkyl radical is C₁-C₄ and preferably denotes methyl, ethyl or propyl. Such polymers are described in particular in FR 1 583 363.
   Among these derivatives, mention may be made more particularly of the adipic acid/dimethylaminohydroxypropyl/diethylenetriamine polymers sold under the name Cartaretine F, F4 or F8 by the company Sandoz.
(6) Polymers obtained by reaction of a polyalkylene polyamine containing two primary amine groups and at least one secondary amine group with a dicarboxylic acid chosen from diglycolic acid and saturated C₃-C₈ aliphatic dicarboxylic acids. The mole ratio of the polyalkylene polyamine to the dicarboxylic acid is between 0.8:1 and 1.4:1; the polyaminoamide resulting therefrom being reacted with epichlorohydrin in a mole ratio of epichlorohydrin relative to the secondary amine group of the polyaminoamide of between 0.5:1 and 1.8:1. Such polymers are described in particular in US 3 227 615 and US 2 961 347.
   Polymers of this type are sold in particular under the name Hercosett 57, PD 170 or Delsette 101 by the company Hercules.
(7) Cyclopolymers of alkyldiallylamine or of dialkyldiallylammonium, such as homopolymers or copolymers, preferably homopolymers, containing, as main constituent of the chain, units corresponding to formula (V) or (VI): 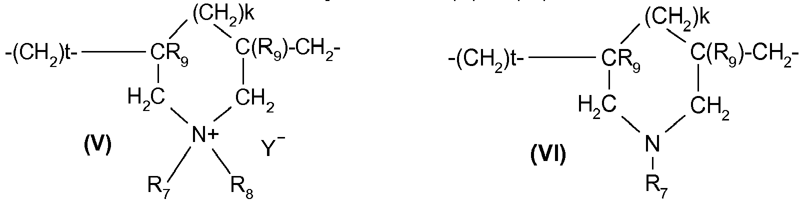 in which formulae k and t are equal to 0 or 1, the sum k + t being equal to 1; R₉ denotes a hydrogen atom or a methyl radical; R₇ and R₈, independently of each other, denote a C₁-C₈ alkyl group, a hydroxyalkyl group in which the alkyl group is C₁-C₅, an amidoalkyl group in which the alkyl is C₁-C₄; R₇ and R₈ can also denote, together with the nitrogen atom to which they are attached, a heterocyclic group such as piperidyl or morpholinyl; R₇ and R₈, independently of each other, preferably denote a C₁-C₄ alkyl group; Y⁻ is an organic or mineral anion such as bromide, chloride, acetate, borate, citrate, tartrate, bisulfate, bisulfite, sulfate or phosphate. These polymers are described in particular in FR 2 080 759 and FR 2 190 406.
   The cyclopolymers preferably comprise at least one unit of formula (V).
   As regards the copolymers, they also comprise an acrylamide monomer. Among the polymers defined above, mention may be made more particularly of the dimethyldiallylammonium chloride homopolymer sold under the name Merquat 100 by the company Nalco (and its homologues of low weight-average molecular mass) and the copolymers of diallyldimethylammonium chloride and of acrylamide sold under the name Merquat 550.
(8) The diquaternary ammonium polymer containing repeating units corresponding to the formula: 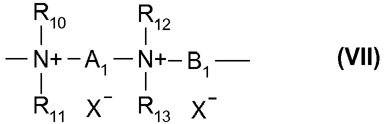 in which formula:
   R₁₀, R₁₁, R₁₂ and R₁₃, which may be identical or different, represent C₁-C₂₀ aliphatic, alicyclic or arylaliphatic radicals or hydroxyalkylaliphatic radicals in which the alkyl radical is C₁-C₄, or alternatively R₁₀, R₁₁, R₁₂ and R₁₃, together or separately, constitute, with the nitrogen atoms to which they are attached, heterocycles optionally containing a second heteroatom other than nitrogen, or alternatively R₁₀, R₁₁, R₁₂ and R₁₃ represent a linear or branched C₁-C₆ alkyl radical substituted with a nitrile, ester, acyl or amide group or a group -CO-O-R₁₄-D or -CO-NH-R₁₄-D where R₁₄ is an alkylene and D is a quaternary ammonium group;
   A₁ and B₁ represent C₂-C₂₀ polymethylene groups which may be linear or branched, and saturated or unsaturated, and which may contain, linked to or intercalated in the main chain, one or more aromatic rings or one or more oxygen or sulfur atoms or sulfoxide, sulfone, disulfide, amino, alkylamino, hydroxyl, quaternary ammonium, ureido, amide or ester groups, and
   X⁻ denotes an anion derived from a mineral or organic acid;
   A₁, R₁₀ and R₁₂ can form, with the two nitrogen atoms to which they are attached, a piperazine ring;
   in addition, if A₁ denotes a linear or branched, saturated or unsaturated alkylene or hydroxyalkylene radical, B₁ can also denote a group -(CH₂)ₙ-CO-D-OC-(CH₂)ₙ- in which n is between 1 and 100 and preferably between 1 and 50, and D denotes:
      a) a glycol residue of formula: -O-Z-O-, where Z denotes a linear or branched hydrocarbon-based radical or a group corresponding to one of the following formulae: -(CH₂-CH₂-O)ₓ-CH₂-CH₂-; -[CH₂-CH(CH₃)-O]_{y}-CH₂-CH(CH₃)-, where x and y denote an integer from 1 to 4, representing a defined and unique degree of polymerization or any number from 1 to 4 representing an average degree of polymerization;
      b) a bis-secondary diamine residue such as a piperazine derivative;
      c) a bis-primary diamine residue of formula: -NH-Y-NH-, where Y denotes a linear or branched hydrocarbon-based radical, or alternatively the radical -CH₂-CH₂-S-S-CH₂-CH₂;
      d) a ureylene group of formula: -NH-CO-NH-.

      Preferably, X⁻ is an anion such as chloride or bromide.
      These polymers have a number-average molecular mass generally of between 1000 and 100 000.
      Polymers of this type are described in particular in FR 2 320 330, FR 2 270 846, FR 2 316 271, FR 2 336 434, FR 2 413 907, US 2 273 780, US 2 375 853, US 2 388 614, US 2 454 547, US 3 206 462, US 2 261 002, US 2 271 378, US 3 874 870, US 4 001 432, US 3 929 990, US 3 966 904, US 4 005 193, US 4 025 617, US 4 025 627, US 4 025 653, US 4 026 945 and US 4 027 020. It is more particularly possible to use polymers that consist of repeating units corresponding to formula (VIII) below: 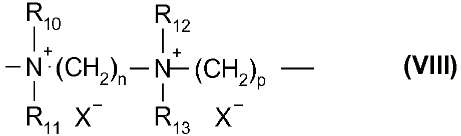 in which R₁₀, R₁₁, R₁₂ and R₁₃, which may be identical or different, denote a C₁-C₄ alkyl or hydroxyalkyl radical, n and p are integers ranging from 2 to 20 approximately, and X⁻ is an anion derived from a mineral or organic acid.
(9) Polyquaternary ammonium polymers consisting of repeating units of formula (IX): 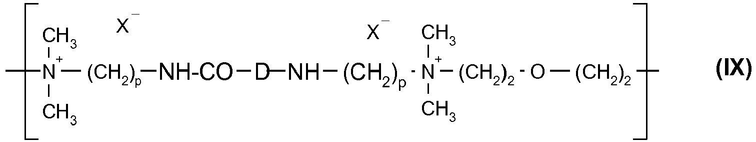 in which p denotes an integer ranging from 1 to 6 approximately, D may be zero or may represent a group -(CH₂)ᵣ-CO- in which r denotes a number equal to 4 or 7, and X⁻ is an anion.
   Such polymers may be prepared according to the processes described in US 4 157 388, US 4 702 906 and US 4 719 282. They are especially described in patent application EP 122 324.
   Among these polymers, examples that may be mentioned include the products Mirapol A 15, Mirapol AD1, Mirapol AZ1 and Mirapol 175 sold by the company Miranol.
(10) Quaternary polymers of vinylpyrrolidone and of vinylimidazole, for instance the products sold under the names Luviquat FC 905, FC 550 and FC 370 by the company BASF.
(11) Polyamines such as Polyquart H sold by Cognis, referred to under the name polyethylene glycol (15) tallow polyamine in the CTFA dictionary.

Other cationic polymers that may be used in the context of the invention are polyalkyleneimines, in particular polyethyleneimines, polymers containing vinylpyridine or vinylpyridinium units, condensates of polyamines and of epichlorohydrin, polyquaternary ureylenes and chitin derivatives.

Among all the cationic polymers that may be used in the context of the present invention, it is preferred to use, alone or as mixtures, polymers of families (1), (2), (3), (7) and (8). In accordance with a more particular embodiment of the invention, it is preferred to use polymers of families (1), (2), (7) and (8).

According to an even more advantageous embodiment of the invention, use is made of polymers of families (1), (7) and (8), alone or as mixtures.

Generally, the content of cationic polymer(s) represents from 0.01% to 20% by weight and more particularly from 0.1% to 10% by weight relative to the weight of the composition.

### Amphoteric polymer

The amphoteric (or zwitterionic) polymers that may be used in accordance with the invention may be chosen from polymers comprising units B and C statistically distributed in the polymer chain, where B denotes a unit derived from a monomer comprising at least one basic nitrogen atom and C denotes a unit derived from an acid monomer comprising one or more carboxylic or sulfonic groups, or alternatively B and C may denote groups derived from carboxybetaine or sulfobetaine zwitterionic monomers;
B and C may also denote a cationic polymer chain comprising primary, secondary, tertiary or quaternary amine groups, in which at least one of the amine groups bears a carboxylic or sulfonic group connected via a hydrocarbon-based radical, or alternatively B and C form part of a chain of a polymer comprising an α,β-dicarboxylic ethylene unit in which one of the carboxylic groups has been made to react with a polyamine comprising one or more primary or secondary amine groups.

The amphoteric polymers corresponding to the definition given above that are more particularly preferred are chosen from the following polymers:
(1) polymers comprising, as monomers, at least one monomer derived from a vinyl compound bearing a carboxylic group such as, more particularly, acrylic acid, methacrylic acid, maleic acid, α-chloroacrylic acid, and at least one basic monomer derived from a substituted vinyl compound containing at least one basic atom chosen in particular from:
   a) dialkylaminoalkyl methacrylates, dialkylaminoalkyl acrylates, dialkylaminoalkylmethacrylamides and dialkylaminoalkylacrylamides. Such compounds are described in US patent No. 3 836 537;
   b) trialkylaminoalkyl methacrylate salts, trialkylaminoalkyl acrylate salts, trialkylaminoalkylmethacrylamide salts and trialkylaminoalkylacrylamide salts.
   Mention may be made especially of the acrylic acid/acrylamidopropyltrimethylammonium chloride copolymer sold by the company Stockhausen under the name Polymer W3794. Mention may also be made of the acrylic acid/acrylamidopropyltrimethylammonium chloride/acrylamide copolymers sold by the company Nalco under the names Merquat 2001 and Merquat 2003;
(2) polymers comprising units derived from:
   a) at least one monomer chosen from acrylamides and methacrylamides substituted on the nitrogen with an alkyl radical,
   b) at least one acidic comonomer containing one or more reactive carboxylic groups, and
   c) at least one basic comonomer such as esters containing primary, secondary, tertiary and quaternary amine substituents of acrylic and methacrylic acids and the product of quaternization of dimethylaminoethyl methacrylate with dimethyl or diethyl sulfate.

   The N-substituted acrylamides or methacrylamides that are more particularly preferred according to the invention are groups in which the alkyl radicals contain from 2 to 12 carbon atoms and more particularly N-ethylacrylamide, N-tert-butylacrylamide, N-tert-octylacrylamide, N-octylacrylamide, N-decylacrylamide, N-dodecylacrylamide and the corresponding methacrylamides.
   The acidic comonomers are chosen more particularly from acrylic acid, methacrylic acid, crotonic acid, itaconic acid, maleic acid and fumaric acid and alkyl monoesters, containing 1 to 4 carbon atoms, of maleic or fumaric acids or anhydrides.
   The preferred basic comonomers are aminoethyl, butylaminoethyl, N,N'-dimethylaminoethyl and N-tert-butylaminoethyl methacrylates. The copolymers whose CTFA (4th edition, 1991) name is Octylacrylamide/acrylates/butylaminoethyl methacrylate copolymer, such as the products sold under the name Amphomer LV by the company National Starch, are particularly used;
(3) copolymers comprising, as monomers, at least one monomer derived from a vinyl compound bearing a carboxylic group, such as, more particularly, acrylic acid, methacrylic acid, maleic acid, α-chloroacrylic acid, and at least one monomer of diallyldialkylammonium salt type, the alkyl groups containing from 1 to 6 carbon atoms. Preferably, the alkyl group is a methyl group.
   Among these polymers, the copolymers comprising, as monomers, dimethyldiallylammonium chloride and acrylic acid, optionally combined with acrylamide, are particularly preferred. Mention may in particular be made of the compounds provided by the company Nalco under the names Merquat 280, Merquat 295, Merquat 3330, Merquat 3331 and Merquat 3333;
(4) crosslinked and alkylated polyamino amides partially or totally derived from polyamino amides of general formula:

   -⁅CO-R₁₀-CO-Z⁆- **(I')**

   in which R₁₀ represents a divalent radical derived from a saturated dicarboxylic acid, a mono- or dicarboxylic aliphatic acid containing an ethylenic double bond, an ester of a lower alkanol containing 1 to 6 carbon atoms of these acids, or a radical derived from the addition of any one of the said acids to a bis(primary) or bis(secondary) amine, and Z denotes a radical from a bis(primary), mono- or bis(secondary) polyalkylene-polyamine and preferably represents:
   a) in proportions of from 60 to 100 mol%, the radical 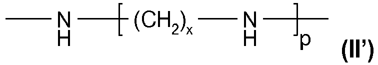
      where x = 2 and p = 2 or 3, or alternatively x = 3 and p = 2,
      this radical being derived from diethylenetriamine, from triethylenetetramine or from dipropylenetriamine;
   b) in proportions of from 0 to 40 mol%, the radical (II') above in which x = 2 and p = 1 and which is derived from ethylenediamine, or the radical derived from piperazine: 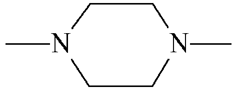
   c) in proportions of from 0 to 20 mol%, the radical -NH(CH₂)₆-NH- derived from hexamethylenediamine, these polyamino amines being crosslinked by addition of a difunctional crosslinking agent chosen from epihalohydrins, diepoxides, dianhydrides and bis-unsaturated derivatives, using from 0.025 to 0.35 mol of crosslinking agent per amine group of the polyamino amide and alkylated by the action of acrylic acid, chloroacetic acid or an alkane sultone, or salts thereof.

   The saturated carboxylic acids are preferably chosen from acids containing 6 to 10 carbon atoms, such as adipic acid, 2,2,4-trimethyladipic acid and 2,4,4-trimethyladipic acid, terephthalic acid, and acids containing an ethylenic double bond, for instance acrylic acid, methacrylic acid and itaconic acid.
   The alkane sultones used in the alkylation are preferably propane sultone or butane sultone, the salts of the alkylating agents are preferably the sodium or potassium salts;
(5) polymers comprising zwitterionic units of formula: 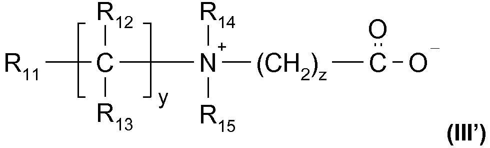 in which R₁₁ denotes a polymerizable unsaturated group such as an acrylate, methacrylate, acrylamide or methacrylamide group, y and z represent an integer from 1 to 3, R₁₂ and R₁₃ represent a hydrogen atom, methyl, ethyl or propyl, R₁₄ and R₁₅ represent a hydrogen atom or an alkyl radical such that the sum of the carbon atoms in R₁₄ and R₁₅ does not exceed 10.
   The polymers comprising such units may also comprise units derived from non-zwitterionic monomers such as dimethyl- or diethylaminoethyl acrylate or methacrylate or alkyl acrylates or methacrylates, acrylamides or methacrylamides or vinyl acetate.
   By way of example, mention may be made of the methyl methacrylate/methyl dimethylcarboxymethylammonioethyl methacrylate copolymer such as the product sold under the name Diaformer Z301 by the company Sandoz;
(6) polymers derived from chitosan comprising monomer units corresponding to the following formulae: 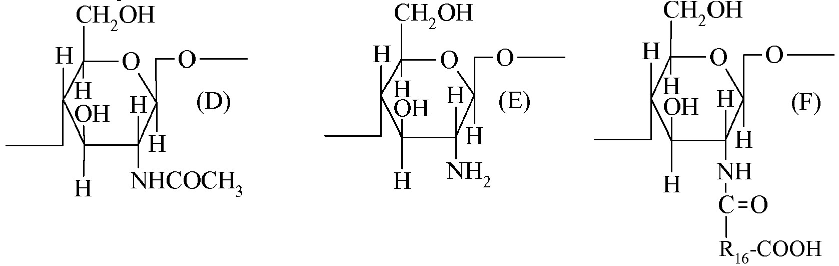 the unit D being present in proportions of between 0 and 30%, the unit E in proportions of between 5% and 50% and the unit F in proportions of between 30% and 90%, it being understood that, in this unit F, R₁₆ represents a radical of formula: 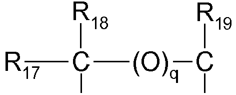
   in which, if q = 0, R₁₇, R₁₈ and R₁₉, which may be identical or different, each represent a hydrogen atom, a methyl, hydroxyl, acetoxy or amino residue, a monoalkylamine residue or a dialkylamine residue that are optionally interrupted with one or more nitrogen atoms and/or optionally substituted with one or more amine, hydroxyl, carboxyl, alkylthio or sulfonic groups, or an alkylthio residue in which the alkyl group bears an amino residue, at least one of the radicals R₁₇, R₁₈ and R₁₉ being, in this case, a hydrogen atom;
   or, if q = 1, R₁₇, R₁₈ and R₁₉ each represent a hydrogen atom, and also the salts formed by these compounds with bases or acids;
(7) polymers derived from the N-carboxyalkylation of chitosan, such as N-carboxymethyl chitosan or N-carboxybutyl chitosan, sold under the name Evalsan powder by the company Jan Dekker;
(8) polymers containing units corresponding to the general formula (IV') are described in French patent 1 400 366: 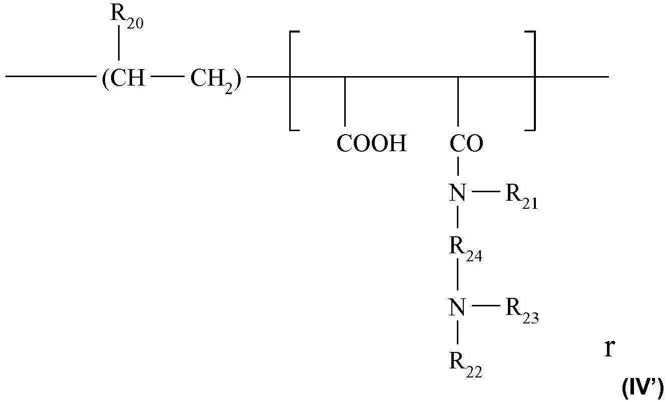 in which R₂₀ represents a hydrogen atom, a CH₃O, CH₃CH₂O or phenyl radical, R₂₁ denotes hydrogen or a lower alkyl radical such as methyl or ethyl, R₂₂ denotes hydrogen or a lower alkyl radical such as methyl or ethyl, R₂₃ denotes a lower alkyl radical such as methyl or ethyl or a radical corresponding to the formula: -R₂₄-N(R₂₂)₂, R₂₄ representing a group -CH₂-CH₂-, -CH₂-CH₂-CH₂-, -CH₂-CH(CH₃)-, R₂₂ having the meanings mentioned above, and also the higher homologues of these radicals, containing up to 6 carbon atoms;
(9) amphoteric polymers of the -D-X-D-X type chosen from:
   a) polymers obtained by the action of chloroacetic acid or sodium chloroacetate on compounds comprising at least one unit of formula:

      -D-X-D-X-D- **(V')**

      where D denotes a radical 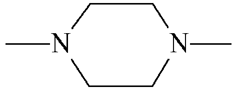 and X denotes the symbol E or E', E or E', which may be identical or different, denote a divalent radical which is an alkylene radical with a straight or branched chain containing up to 7 carbon atoms in the main chain, which is unsubstituted or substituted with hydroxyl groups and which can comprise, in addition to oxygen, nitrogen and sulfur atoms, 1 to 3 aromatic and/or heterocyclic rings; the oxygen, nitrogen and sulfur atoms being present in the form of ether, thioether, sulfoxide, sulfone, sulfonium, alkylamine or alkenylamine groups, hydroxyl, benzylamine, amine oxide, quaternary ammonium, amide, imide, alcohol, ester and/or urethane groups;
   b) polymers of formula:

      -D-X-D-X- **(VI')**

      where D denotes a radical 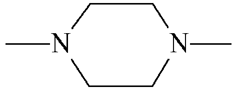 and X denotes the symbol E or E' and at least once E'; E having the meaning given above and E' is a divalent radical which is an alkylene radical with a straight or branched chain containing up to 7 carbon atoms in the main chain, which is unsubstituted or substituted with one or more hydroxyl radicals and containing one or more nitrogen atoms, the nitrogen atom being substituted with an alkyl chain that is optionally interrupted with an oxygen atom and necessarily comprising one or more carboxyl functions or one or more hydroxyl functions and betainized by reaction with chloroacetic acid or sodium chloroacetate;
(10) (C₁-C₅)alkyl vinyl ether/maleic anhydride copolymers partially modified by semiamidation with an N,N-dialkylaminoalkylamine such as N,N-dimethylaminopropylamine or by semiesterification with an N,N-dialkanolamine. These copolymers can also comprise other vinyl comonomers such as vinylcaprolactam, and mixtures thereof.

The amphoteric polymers that are particularly preferred according to the invention are those of family (3). Mention may be made in particular of copolymers comprising, as monomers, dimethyldiallylammonium chloride and acrylic acid, optionally combined with acrylamide.

In the composition of the invention, the amphoteric polymer(s) preferably represent(s) from 0.01% to 20% and better still from 0.1% to 10% by weight relative to the total weight of the composition.

Preferably, the weight ratio of dicarbonyl derivatives corresponding to formula (I) and/or derivatives thereof and/or hydrate forms thereof and/or salts thereof to cationic or cationic and amphoteric polymer(s) ranges from 1 to 50 and better still from 2 to 20.

The composition according to the invention may also comprise at least one cellulose-based polymer,
According to the invention, the term "cellulose-based" polymer means any polysaccharide compound having in its structure sequences of glucose residues linked together via β-1,4 bonds; in addition to unsubstituted celluloses, the cellulose derivatives may be anionic, cationic, amphoteric or nonionic. Thus, the cellulose polymers of the invention may be chosen from unsubstituted celluloses, including those in a microcrystalline form, and cellulose ethers. Among these cellulose polymers, cellulose ethers, cellulose esters and cellulose ester ethers are distinguished. Among the cellulose esters are mineral cellulose esters (cellulose nitrates, sulfates, phosphates, etc.), organic cellulose esters (cellulose monoacetates, triacetates, amidopropionates, acetatebutyrates, acetatepropionates and acetatetrimellitates, etc.), and mixed organic/mineral cellulose esters, such as cellulose acetatebutyrate sulfates and cellulose acetatepropionate sulfates. Among the cellulose ester ethers, mention may be made of hydroxypropylmethylcellulose phthalates and ethylcellulose sulfates.

According to a particular embodiment, the composition comprises at least one amphoteric or zwitterionic surfactant.

In particular, the amphoteric or zwitterionic surfactant(s), which are preferably non-silicone, which may be used in the present invention may especially be derivatives of optionally quaternized aliphatic secondary or tertiary amines, in which derivatives the aliphatic group is a linear or branched chain comprising from 8 to 22 carbon atoms, the said amine derivatives containing at least one anionic group, for instance a carboxylate, sulfonate, sulfate, phosphate or phosphonate group.

Mention may be made in particular of (C₈-C₂₀)alkylbetaines, (C₈-C₂₀)alkylsulfobetaines, (C₈-C₂₀)alkylamido(C₃-C₈)alkylbetaines and (C₈-C₂₀)alkylamido(C₆-C₈)alkylsulfobetaines.

Among the optionally quaternized secondary or tertiary aliphatic amine derivatives that may be used, as defined above, mention may also be made of the compounds of respective structures (**B1**) and (**B2**) below:

Rₐ-C(O)-NH-CH₂-CH₂-N⁺(R_{b})(R_{c})-CH₂C(O)O⁻, M⁺, X⁻ **(B1)**

in which formula:
▪ Rₐ represents a C₁₀-C₃₀ alkyl or alkenyl group derived from an acid Rₐ-COOH preferably present in hydrolysed coconut oil, or a heptyl, nonyl or undecyl group;
▪ R_{b} represents a β-hydroxyethyl group; and
▪ R_{c} represents a carboxymethyl group;
▪ M⁺ represents a cationic counterion derived from an alkali metal or alkaline-earth metal, such as sodium, an ammonium ion or an ion derived from an organic amine, and
▪ X⁻ represents an organic or mineral anionic counterion, such as that chosen from halides, acetates, phosphates, nitrates, (C₁-C₄)alkyl sulfates, (C₁-C₄)alkyl- or (C₁-C₄)alkylarylsulfonates, in particular methyl sulfate and ethyl sulfate; or alternatively M⁺ and X⁻ are absent;

R_{a'}-C(O)-NH-CH₂-CH₂-N(B)(B') **(B2)**

in which formula:
▪ B represents the group -CH₂-CH₂-O-X';
▪ B' represents the group -(CH₂)_{z}Y', with z = 1 or 2;
▪ X' represents the group -CH₂-C(O)OH, -CH₂-C(O)OZ', -CH₂-CH₂-C(O)OH, -CH₂-CH₂-C(O)OZ', or a hydrogen atom;
▪ Y' represents the group -C(O)OH, -C(O)OZ', -CH₂-CH(OH)-SO₃H or the group -CH₂-CH(OH)-SO₃-Z';
▪ Z' represents a cationic counterion derived from an alkali metal or alkaline-earth metal, such as sodium, an ammonium ion or an ion derived from an organic amine;
▪ R_{a'} represents a C₁₀-C₃₀ alkyl or alkenyl group of an acid R_{a'}-C(O)OH preferably present in hydrolysed linseed oil or coconut oil, an alkyl group, especially of C₁₇ and its iso form, or an unsaturated C₁₇ group.

The compounds of this type are classified in the CTFA dictionary, 5th edition, 1993, under the names disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid and cocoamphodipropionic acid.

By way of example, mention may be made of the cocoamphodiacetate sold by the company Rhodia under the trade name Miranol® C2M Concentrate.

Use may also be made of compounds of formula (B'2):

**Ra"-NH-CH(Y")-(CH₂)n-C(O)-NH-(CH₂)n'-N(Rd)(Re)** **(B'2)**

in which formula:
▪ Y" represents the group -C(O)OH, -C(O)OZ", -CH₂-CH(OH)-SO₃H or the group -CH₂-CH(OH)-SO₃-Z";
▪ Rd and Re, independently of each other, represent a C₁-C₄ alkyl or hydroxyalkyl radical;
▪ Z" represents a cationic counterion derived from an alkali metal or alkaline-earth metal, such as sodium, an ammonium ion or an ion derived from an organic amine;
▪ Ra" represents a C₁₀-C₃₀ alkyl or alkenyl group of an acid Ra"-C(O)OH preferably present in coconut oil or in hydrolysed linseed oil;
▪ n and n' denote, independently of each other, an integer ranging from 1 to 3.

Among the compounds of formula (B'2), mention may be made of the compound classified in the CTFA dictionary under the name sodium diethylaminopropyl cocoaspartamide and sold by the company Chimex under the name Chimexane HB.

In accordance with a particular embodiment of the invention, the content of amphoteric or zwitterionic surfactant(s), when they are present, ranges from 0.05% to 30% by weight, preferably from 0.5% to 10% by weight and more preferably from 0.1% to 5% by weight, relative to the total weight of the composition.

The compositions according to the invention may be provided in any galenical form conventionally used and in particular in the form of an aqueous, alcoholic or aqueous/alcoholic solution or suspension or oily solution or suspension; a solution or a dispersion of the lotion or serum type; an emulsion, in particular of liquid or semi-liquid consistency, of the O/W, W/O or multiple type; a suspension or emulsion of soft consistency of (O/W) or (W/O) cream type; an aqueous or anhydrous gel, or any other cosmetic form.

These compositions may be packaged in pump-action bottles or in aerosol containers, so as to apply the composition in vaporized (lacquer) form or in the form of a mousse. Such packaging forms are indicated, for example, when it is desired to obtain a spray or a mousse, for the treatment of the hair. In these cases, the composition preferably comprises at least one propellant.

The compositions of the invention may be aqueous or anhydrous. They are preferably aqueous and then comprise water at a concentration ranging from 5% to 98%, better still from 5% to 90% and even better still from 10% to 90% by weight relative to the total weight of the composition.

The composition may in particular comprise one or more organic solvents, in particular water-soluble solvents, such as C₁-C₇ alcohols; mention may in particular be made of C₁-C₇ aliphatic monoalcohols, C₆-C₇ aromatic monoalcohols, C₃-C₇ polyols or C₃-C₇ polyol ethers, which may be employed alone or as a mixture with water.

The composition of the invention may also comprise at least one common cosmetic ingredient, chosen in particular from propellants; oils; solid fatty substances and in particular C₈-C₄₀ esters; C₈-C₄₀ acids; C₈-C₄₀ alcohols; surfactants other than those described previously; sunscreens; moisturizers; antidandruff agents; antioxidants; chelating agents; nacreous agents and opacifiers; plasticizers or coalescers; fillers; silicones and in particular polydimethylsiloxanes; polymeric or non-polymeric thickeners; gelling agents; emulsifiers; polymers, in particular conditioning or styling polymers other than those described previously; fragrances; basifying agents such as sodium hydroxide or acidifying agents; silanes; crosslinking agents. Needless to say, the composition may comprise several cosmetic ingredients featured in the above list.

Depending on their nature and the intended use of the composition, the common cosmetic ingredients may be present in usual amounts which may be readily determined by a person skilled in the art and which may be, for each ingredient, between 0.01% and 80% by weight. A person skilled in the art will take care to choose the ingredients included in the composition, and also the amounts thereof, such that they do not harm the properties of the compositions of the present invention.

The pH of the composition is preferably less than 4, and preferentially ranges from 1 to 3, better still from 1.5 to 3 and even better still from 1.7 to 3.

It may be adjusted to the desired value by means of acidifying and/or basifying agents usually used for treating keratin fibres.

The basifying agent may be chosen from mineral or organic or hybrid alkaline agents, or mixtures thereof.

The mineral alkaline agent(s) are preferably chosen from aqueous ammonia, alkali metal carbonates or bicarbonates such as sodium or potassium carbonate and sodium or potassium bicarbonate, sodium hydroxide or potassium hydroxide, or mixtures thereof.

The organic alkaline agent(s) are preferably chosen from organic amines with a pKb at 25°C of less than 12, preferably less than 10 and even more advantageously less than 6. It should be noted that it is the pKb corresponding to the function of highest basicity.

Mention may be made, as hybrid compounds, of the salts of the abovementioned amines with acids, such as carbonic acid or hydrochloric acid.

The organic alkaline agent(s) are chosen, for example, from amine derivatives such as alkanolamines, oxyethylenated and/or oxypropylenated ethylenediamines, amino acids of amines such as a 1,3-diaminopropane, 1,3-diamino-2-propanol, spermine or spermidine.

The term "alkanolamine" means an organic amine comprising a primary, secondary or tertiary amine function, and one or more linear or branched C₁-C₈ alkyl groups bearing one or more hydroxyl radicals.

Sodium hydroxide is in particular suitable for use in the invention.

The acidifying agent may be chosen from mineral or organic acids, for instance hydrochloric acid, phosphoric acid or lactic acid.

The composition according to the invention is preferably in the form of styling or care gels, care lotions or creams, conditioners, masks or sera.

The composition according to the invention may be obtained by mixing several compositions.

The process of the invention comprises the application of the composition described previously, followed by a step of straightening the hair with an iron, preferably at a temperature of at least 150°C. The straightening iron is known in the prior art. It consists in straightening the hair with flat heating tongs, which are generally metallic. The straightening irons are generally used at a temperature ranging from 150 to 250°C.

The process of the invention may comprise other intermediate steps aimed at improving the straightening of the hair.

According to a particular embodiment, the process of the invention comprises the application of the composition of the invention to dry hair, and a time of contact of the composition with the hair ranging from 10 to 60 minutes and preferably from 20 to 40 minutes. After this leave-on time, straightening with a brush and with a hairdryer (blow-drying) is performed. The hair is then straightened with a straightening iron at a temperature ranging from 150 to 250°C and preferably from 210 to 230°C.

The process of the invention may comprise the application of other hair agents as a pretreatment or post-treatment. In particular, it may comprise the application of a conditioning care product as a post-treatment.

According to another embodiment, the hair straightening process comprises a step of washing the hair and then of drying with a hairdryer before applying the composition of the invention. According to this particular embodiment, the steps described above are then encountered, such as the contact time of the composition, the straightening with a straightening iron, the application of a conditioning agent and the rinsing, all these steps possibly being performed independently of each other, blow-drying possibly being intercalated between the contact of the composition according to the invention and the straightening with the iron. According to a particular embodiment, the straightening with the straightening iron is performed in several passes on the hair, in general 8 to 10 passes.

The process of the present invention is preferably performed without a step of permanent reshaping at basic pH or based on a reducing agent.

### Example 1

The following composition is prepared. The amounts are indicated as weight percentages of starting material in unmodified form relative to the total weight of the composition.

| Composition | **% by weight** |
|---|---|
| Hydroxypropylmethylcellulose (Methocel F4M from Dow Chemical) | 1 |
| Glyoxylic acid (50% aqueous solution) | 16 |
| Disodium cocoamphodiacetate (sodium N-cocoylamidoethyl-N-ethoxycarboxylmethyl glycinate) 30% aqueous solution (Miranol C2M Conc. NP from Rhodia) | 2 |
| Polyquaternium-37 (and) propylene glycol dicaprylate/dicaprate (and) PPG-1 Trideceth-6 (Salcare SC 96 from BASF) | 2.5 |
| Lactic acid | 2.5 |
| Sodium hydroxide (10% aqueous solution) | 7 |
| Bisamino PEG/PPG-41-3 aminoethyl PG-propyl dimethicone at 30% AM in dipropylene glycol (Silsoft A 843 from Momentive Performance Materials) | 2 |
| Water | qs |

| | |
|---|---|
| *pH 2.2 ± 0.2 | |

The above composition is applied to a lock of hair having a substantial level of curliness. After a leave-on time of 20 minutes on the hair, blow-drying is performed with a hairdryer and the lock is then straightened using a straightening iron. The evolution of fumes during the use of the iron is low.

The lock is then washed, and then dried with a hairdryer. A straight lock of hair free of curliness is thus obtained.

### Other examples

The following compositions are prepared, compositions C1 to C6 being comparative examples and compositions 2 to 6 being compositions in accordance with the invention.

| | C1 | C2 | C3 | C4 | C5 | C6 | 2 | 3 | 4 | 5 | 6 |
|---|---|---|---|---|---|---|---|---|---|---|---|
| Glyoxylic acid | 5 g | | | | | | 5 g | 5 g | 5 g | | |
| Pyruvic acid | | 8 g | | | | | | | | 8 g | |
| Ketoglutaric acid | | | 8 g | | | | | | | | 8 g |
| Polyquaternium 6 | | | | 1 g | | | 1 g | | | 1 g | |
| Polyquaternium 10 | | | | | 1 g | | | 1 g | | | |
| Salcare SC96 | | | | | | 3 g | | | 3 g | | 3 g |
| Water qs 100 g | x | x | x | x | x | x | x | x | x | x | x |
| Sodium hydroxide qs pH 2.2 | x | x | x | x | x | x | x | x | x | x | x |

### Process for a one-step use:

The compositions, optionally shaken before use, are applied to curly hair, which may be natural or dyed, or sensitized by a prior bleaching step at a rate of 1 g per 2 g of hair. After 15 minutes, the hair is rinsed, dried with a hairdryer (blow-drying) and then straightened by treating with flat tongs heated to 210°C. It is subsequently shampooed to examine the remanence of the straightening effects and of modification of the mechanical and cosmetic properties of the fibres.

Compositions 2, 3 and 4 make it possible to obtain straightening properties superior to those obtained with compositions C1 and C4 to C6, whether in terms of: curl relaxation, protection of the natural or artificial colour, resistance of the fibres to mechanical stresses (pulling, rubbing, twisting), sheen, smooth feel and smooth look.

Similarly, composition 5 gives performance superior to that obtained with composition C2 or C4.

Similarly, composition 6 gives performance superior to that obtained with composition C3 or C6.

### Comparative example:

Compositions A and B were prepared with the following compositions (active material in wt %).

| Composition | A (invention) (% wt/wt) | Composition B (Comparative) (%wt/wt) |
|---|---|---|
| GLYOXYLIC ACID | 5 am | - |
| PROLISS 100 (POLYTECHNO INDUSTRIAS QUIMICAS) | - | 5 am |
| POLYQUATERNIUM-67 | 0.95 am | 0.95 am |
| DEIONIZED WATER | Qs 100 | Qs 100 |

2.7g hair locks of curl type IV bleached hair were washed with a shampoo and blow dried. Then 2.7g of composition A was applied to one of the hair lock and 2.7g of composition B was applied to another hair lock. After a leave-on time of 20 minutes on the hair, the locks were blow-dried with a hairdryer (brushing with 15 passes of a brush) and were then straightened with a straightening iron (10 passes). The locks were then washed with a shampoo and let air dried (spontaneous). Then, the hair locks were washed again with a second shampoo and then let air dried (spontaneous). Better lasting curl reduction was observed on the hair lock treated with composition A.

## Claims

1. Cosmetic composition comprising at least one cationic polymer or at least one cationic polymer and one amphoteric polymer and at least 3% by weight relative to the total weight of the composition of one or more dicarbonyl compounds corresponding to formula (I) below and/or hydrates thereof and/or salts thereof: 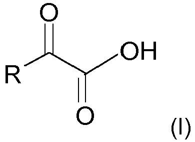 in which formula (I):
R represents an atom or group chosen from i) hydrogen, ii) carboxyl -C(O)OH, iii) linear or branched C₁-C₆ alkyl which is optionally substituted, preferably with at least one hydroxyl -OH radical, carboxyl -C(O)-OH radical or halogen radical such as Br; iv) optionally substituted phenyl, v) optionally substituted benzyl, iv) and v) preferably being optionally substituted with at least one -OH or -C(O)OH radical; vi) an indolyl radical and vii) an imidazolylmethyl radical and tautomers thereof such as 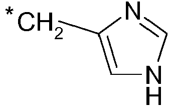 with * representing the part linked to the rest of the molecule.

2. Composition according to Claim 1, in which R represents i) a hydrogen atom or ii) a linear or branched C₁-C₆ alkyl group optionally substituted with a carboxyl group.

3. Composition according to either of the preceding claims, in which the dicarbonyl compound(s) corresponding to formula (I) and/or hydrates thereof and/or salts thereof are chosen from glyoxylic acid and pyruvic acid, salts thereof and hydrates thereof, preferably from glyoxylic acid and the hydrate forms of this compound.

4. Composition according to Claim 3, in which the glyoxylic acid is in its hydrate form.

5. Composition according to any one of Claims 1 to 4, comprising from 3% to 15% by weight of one or more dicarbonyl compounds corresponding to formula (I) and/or hydrates thereof and/or salts thereof, preferably from 5% to 10% by weight relative to the total weight of the composition.

6. Composition according to any one of Claims 1 to 5, in which the cationic polymer(s) are chosen from the following polymers, alone or as mixtures:
(1) Homopolymers or copolymers derived from acrylic or methacrylic esters or amides and comprising at least one of the units of formula (I), (II), (III) or (IV) below: 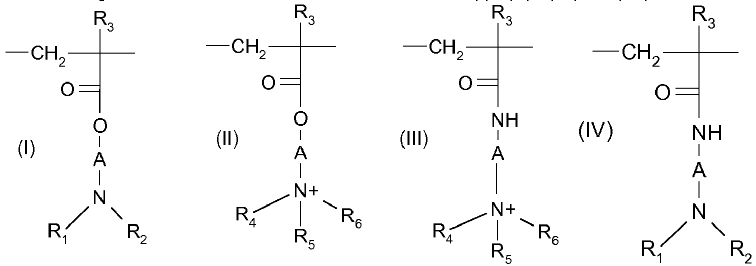 in which:
R₃, which may be identical or different, denotes a hydrogen atom or a CH₃ radical;
A, which may be identical or different, represents a linear or branched C₁-C₆ alkyl group or a hydroxyalkyl group, the alkyl of which is C₁-C₄;
R₄, R₅ and R₆, which may be identical or different, represent a C₁-C₁₈ alkyl group or a benzyl radical;
R₁ and R₂, which may be identical or different, represent hydrogen or a C₁-C₆ alkyl group;
(2) Cationic cellulose derivatives;
(3) Cationic guar gums;
(4) Polymers consisting of piperazinyl units and of linear or branched divalent alkyl or hydroxyalkyl radicals, optionally interrupted with oxygen, sulfur or nitrogen atoms or with aromatic or heterocyclic rings, and also the oxidation and/or quaternization products of these polymers;
(5) Water-soluble polyaminoamides which are optionally crosslinked;
(6) Polymers obtained by reaction of a polyalkylene polyamine containing two primary amine groups and at least one secondary amine group with a dicarboxylic acid;
(7) Cyclopolymers of alkyldiallylamine or of dialkyldiallylammonium, in the form of homopolymers or copolymers containing, as main constituent of the chain, units corresponding to formula (V) or (VI): 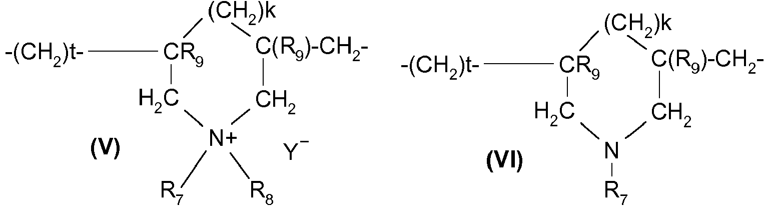 in which formulae k and t are equal to 0 or 1, the sum k + t being equal to 1; R₉ denotes a hydrogen atom or a methyl radical; R₇ and R₈, independently of each other, denote a C₁-C₈ alkyl group, a hydroxyalkyl group in which the alkyl group is C₁-C₅, an amidoalkyl group in which the alkyl is C₁-C₄; R₇ and R₈ can also denote, together with the nitrogen atom to which they are attached, a heterocyclic group; Y⁻ is an organic or mineral anion;
(8) The diquaternary ammonium polymers containing repeating units corresponding to the formula: 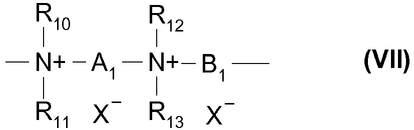 in which formula (VII):
R₁₀, R₁₁, R₁₂ and R₁₃, which may be identical or different, represent linear, branched or cyclic, saturated, unsaturated or aromatic, C₁-C₂₀ hydrocarbon-based radicals, linear or branched hydroxyalkyl radicals in which the alkyl part is C₁-C₄, linear or branched C₁-C₆ alkyl radicals, substituted with a nitrile, ester, acyl or amide group or -CO-O-R₁₄-D or - CO-NH-R₁₄-D with R₁₄ representing an alkyl radical and D a quaternary ammonium group, or form, together or separately, with the nitrogen atoms to which they are attached, heterocycles optionally containing a second heteroatom other than nitrogen;
A₁ and B₁ represent linear or branched, saturated or unsaturated, C₂-C₂₀ radicals, optionally substituted or interrupted with one or more aromatic rings, oxygen or sulfur atoms or groups bearing at least one of these atoms;
X⁻ denotes an organic or mineral anion;
A₁, R₁₀ and R₁₂ can form, with the two nitrogen atoms to which they are attached, a piperazine ring; in addition, if A₁ denotes a linear or branched, saturated or unsaturated alkylene or hydroxyalkylene radical, B₁ can also denote a group -(CH₂)ₙCO-D-OC(CH₂)ₙ- in which n is between 1 and 100, and D denotes a glycol, bis-secondary diamine, bis-primary diamine or ureylene residue;
(9) Polyquaternary ammonium polymers consisting of repeating units of formula (IX): 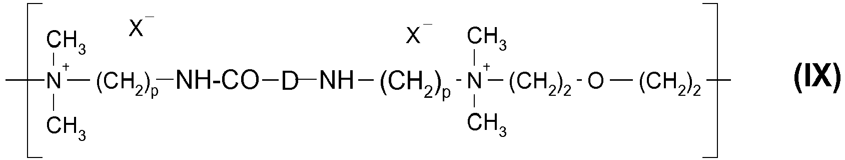 in which p denotes an integer ranging from 1 to 6, D may be zero or may represent a group -(CH₂)ᵣ-CO- in which r denotes a number equal to 4 or 7, and X⁻ is an organic or mineral anion;
(10) Quaternary polymers of vinylpyrrolidone and of vinylimidazole;
(11) Polyamines.

7. Composition according to Claim 6, **characterized in that** the cationic polymer(s) are chosen from the polymers (1), (2), (3), (7) and (8), preferably from the polymers (1), (2), (7) and (8) and better still from the polymers (1), (7) and (8), alone or as mixtures.

8. Composition according to any one of Claims 1 to 5, in which the amphoteric polymer(s) are chosen from the following compounds:
(1) polymers comprising, as monomers, at least one monomer derived from a vinyl compound bearing a carboxylic group, preferably acrylic acid, methacrylic acid, maleic acid, α-chloroacrylic acid, and at least one basic monomer derived from a substituted vinyl compound containing at least one basic atom chosen from:
a) dialkylaminoalkyl methacrylates, dialkylaminoalkyl acrylates, dialkylaminoalkylmethacrylamides and dialkylaminoalkylacrylamides.
b) trialkylaminoalkyl methacrylate salts, trialkylaminoalkyl acrylate salts, trialkylaminoalkylmethacrylamide salts and trialkylaminoalkylacrylamide salts;
(2) polymers comprising units derived from:
a) at least one monomer chosen from acrylamides or methacrylamides substituted on the nitrogen with an alkyl radical preferably containing from 2 to 12 carbon atoms;
b) at least one acidic comonomer containing one or more reactive carboxylic groups, preferably chosen from acrylic acid, methacrylic acid, crotonic acid, itaconic acid, maleic acid and fumaric acid and also from alkyl monoesters, containing 1 to 4 carbon atoms, of maleic or fumaric acids or anhydrides;
c) at least one basic comonomer such as esters containing primary, secondary, tertiary and quaternary amine substituents of acrylic and methacrylic acids and the product of quaternization of dimethylaminoethyl methacrylate with dimethyl or diethyl sulfate;
(3) copolymers comprising, as monomers, at least one monomer derived from a vinyl compound bearing a carboxylic group, such as, more particularly, acrylic acid, methacrylic acid, maleic acid, α-chloroacrylic acid, and at least one monomer of diallyldialkylammonium salt type, the alkyl groups containing from 1 to 6 carbon atoms;
(4) crosslinked and alkylated polyamino amides partially or totally derived from polyamino amides of general formula:
-⁅CO-R₁₀-CO-Z⁆- **(I')**
in which R₁₀ represents a divalent radical derived from a saturated dicarboxylic acid, a mono- or dicarboxylic aliphatic acid containing an ethylenic double bond, an ester of a lower alkanol containing 1 to 6 carbon atoms of these acids, or a radical derived from the addition of any one of the said acids to a bis(primary) or bis(secondary) amine, and Z denotes a radical from a bis(primary), mono- or bis(secondary) polyalkylene polyamine and preferably represents:
a) in proportions of from 60 to 100 mol%, the radical 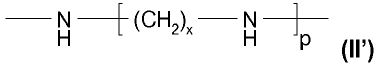
where x = 2 and p = 2 or 3, or alternatively x = 3 and p = 2,
this radical being derived from diethylenetriamine, from triethylenetetramine or from dipropylenetriamine;
b) in proportions of from 0 to 40 mol%, the radical (II') above in which x = 2 and p = 1 and which is derived from ethylenediamine, or the radical derived from piperazine: 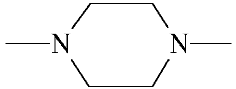
c) in proportions of from 0 to 20 mol%, the radical -NH(CH₂)₆-NH- being derived from hexamethylenediamine, these polyamino amines being crosslinked by addition of a difunctional crosslinking agent chosen from epihalohydrins, diepoxides, dianhydrides and bis-unsaturated derivatives, using from 0.025 to 0.35 mol of crosslinking agent per amine group of the polyamino amide and alkylated by the action of acrylic acid, chloroacetic acid or an alkane sultone, or salts thereof;
(5) polymers comprising zwitterionic units of formula: 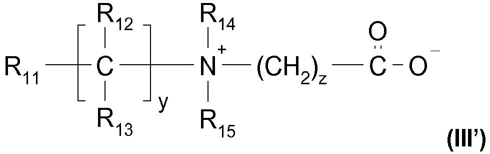 in which R₁₁ denotes a polymerizable unsaturated group such as an acrylate, methacrylate, acrylamide or methacrylamide group, y and z represent an integer from 1 to 3, R₁₂ and R₁₃ represent a hydrogen atom, methyl, ethyl or propyl, R₁₄ and R₁₅ represent a hydrogen atom or an alkyl radical such that the sum of the carbon atoms in R₁₄ and R₁₅ does not exceed 10;
(6) polymers derived from chitosan comprising monomer units corresponding to the following formulae: 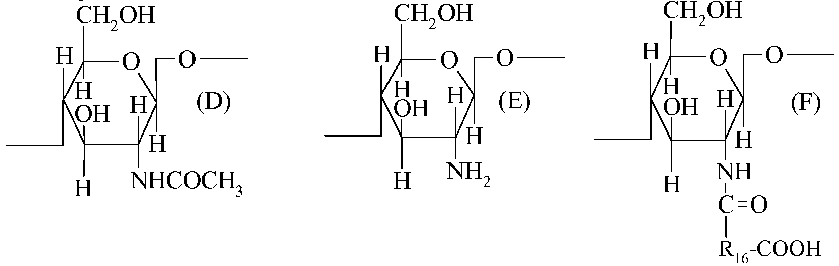 the unit D being present in proportions of between 0 and 30%, the unit E in proportions of between 5% and 50% and the unit F in proportions of between 30% and 90%, it being understood that, in this unit F, R₁₆ represents a radical of formula: 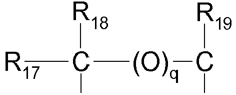
in which, if q = 0, R₁₇, R₁₈ and R₁₉, which may be identical or different, each represent a hydrogen atom, a methyl, hydroxyl, acetoxy or amino residue, a monoalkylamine residue or a dialkylamine residue that are optionally interrupted with one or more nitrogen atoms and/or optionally substituted with one or more amine, hydroxyl, carboxyl, alkylthio or sulfonic groups, or an alkylthio residue in which the alkyl group bears an amino residue, at least one of the radicals R₁₇, R₁₈ and R₁₉ being, in this case, a hydrogen atom;
or, if q = 1, R₁₇, R₁₈ and R₁₉ each represent a hydrogen atom, and also the salts formed by these compounds with bases or acids;
(7) polymers derived from the N-carboxyalkylation of chitosan;
(8) polymers containing units corresponding to the general formula (IV'): 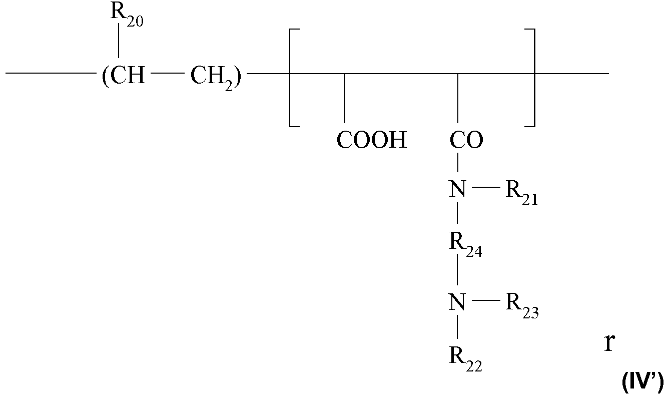
in which R₂₀ represents a hydrogen atom, a CH₃O, CH₃CH₂O or phenyl radical, R₂₁ denotes hydrogen or a lower alkyl radical such as methyl or ethyl, R₂₂ denotes hydrogen or a lower alkyl radical such as methyl or ethyl, R₂₃ denotes a lower alkyl radical such as methyl or ethyl or a radical corresponding to the formula: -R₂₄-N(R₂₂)₂, R₂₄ representing a group -CH₂-CH₂-, -CH₂-CH₂-CH₂- or -CH₂-CH(CH₃)-, R₂₂ having the meanings mentioned above,
and also the higher homologues of these radicals, containing up to 6 carbon atoms;
(9) amphoteric polymers of the -D-X-D-X type chosen from:
a) polymers obtained by the action of chloroacetic acid or sodium chloroacetate on compounds comprising at least one unit of formula:
-D-X-D-X-D- **(V')**
where D denotes a radical 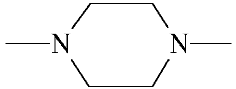 and X denotes the symbol E or E', E or E', which may be identical or different, denote a divalent radical that is an alkylene radical with a straight or branched chain containing up to 7 carbon atoms in the main chain, which is unsubstituted or substituted with hydroxyl groups and which can comprise, in addition to oxygen, nitrogen and sulfur atoms, 1 to 3 aromatic and/or heterocyclic rings; the oxygen, nitrogen and sulfur atoms being present in the form of ether, thioether, sulfoxide, sulfone, sulfonium, alkylamine or alkenylamine groups, hydroxyl, benzylamine, amine oxide, quaternary ammonium, amide, imide, alcohol, ester and/or urethane groups,
b) polymers of formula:
-D-X-D-X- **(VI')**
where D denotes a radical 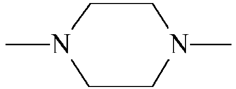 and X denotes the symbol E or E' and at least once E'; E having the meaning given above and E' is a divalent radical that is an alkylene radical with a straight or branched chain containing up to 7 carbon atoms in the main chain, which is unsubstituted or substituted with one or more hydroxyl radicals and containing one or more nitrogen atoms, the nitrogen atom being substituted with an alkyl chain that is optionally interrupted with an oxygen atom and necessarily comprising one or more carboxyl functions or one or more hydroxyl functions and betainized by reaction with chloroacetic acid or sodium chloroacetate;
(10) (C₁-C₅)alkyl vinyl ether/maleic anhydride copolymers partially modified by semiamidation with an N,N-dialkylaminoalkylamine or by semiesterification with an N,N-dialkanolamine;
and mixtures thereof.

9. Composition according to any one of the preceding claims, **characterized in that** the amphoteric polymer(s) are chosen from copolymers comprising as monomers at least one monomer derived from a vinyl compound bearing a carboxylic group, such as acrylic acid, methacrylic acid, maleic acid or α-chloroacrylic acid, and at least one monomer of diallyldialkylammonium salt type, the alkyl groups containing from 1 to 6 carbon atoms, more particularly from copolymers comprising as monomers dimethyldiallylammonium chloride and acrylic acid, optionally combined with acrylamide.

10. Composition according to any one of the preceding claims, in which the content of cationic polymers ranges from 0.01% to 20% by weight and preferably from 0.1% to 10% by weight relative to the total weight of the composition.

11. Composition according to any one of the preceding claims, in which the weight ratio of dicarbonyl compounds corresponding to formula (I) and/or hydrate form thereof and/or salts thereof to cationic or cationic and amphoteric polymer(s) ranges from 1 to 50 and better still from 2 to 20.

12. Composition according to any one of the preceding claims, **characterized in that** it is aqueous and comprises water in a concentration preferably ranging from 5% to 98%, better still from 5% to 90% and even better still from 10% to 90% by weight relative to the total weight of the composition.

13. Composition according to any one of the preceding claims, **characterized in that** it has a pH of less than 4, the pH preferentially ranging from 1 to 3, better still from 1.5 to 3 and even better still from 1.7 to 3.

14. Composition according to any one of the preceding claims, **characterized in that** it results from the mixing of several compositions.

15. Process for straightening keratin fibres, especially the hair, which comprises the application to the hair of the composition according to any one of the preceding claims, preferably followed by a contact time ranging from 10 to 60 minutes and then a straightening step using a straightening iron at a temperature of at least 150°C, preferably ranging from 150 to 250°C.

16. Use of the composition according to any one of Claims 1 to 14, for straightening/relaxing keratin fibres, especially the hair.

## Patentansprüche

1. Kosmetische Zusammensetzung, umfassend mindestens ein kationisches Polymer oder mindestens ein kationisches Polymer und ein amphoteres Polymer und mindestens 3 Gew.-%, bezogen auf das Gesamtgewicht der Zusammensetzung, einer oder mehrerer Dicarbonylverbindungen gemäß nachstehender Formel (I) und/oder Hydrate davon und/oder Salze davon: 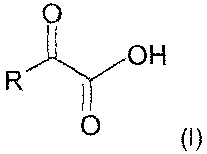 wobei in Formel (I):
R für ein Atom oder eine Gruppe steht, das bzw. die ausgewählt ist aus: i) Wasserstoff, ii) Carboxyl -C(O)OH, iii) linearem oder verzweigtem C₁-C₆-Alkyl, das gegebenenfalls substituiert ist, vorzugsweise durch mindestens einen Hydroxylrest - OH, einen Carboxylrest -C(O)OH oder ein Halogen wie Br; iv) gegebenenfalls substituiertem Phenyl, v) gegebenenfalls substituiertem Benzyl, wobei iv) und v) vorzugsweise gegebenenfalls durch mindestens einen -OH- oder -C(O)OH-Rest substituiert sind; vi) einem Indolylrest und vii) einem Imidazolylmethylrest und Tautomeren davon wie 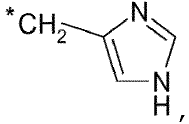 wobei * für den mit dem Rest des Moleküls verknüpften Teil steht.

2. Zusammensetzung nach Anspruch 1, wobei R für i) ein Wasserstoffatom oder ii) eine lineare oder verzweigte C₁-C₆-Alkylgruppe, die gegebenenfalls durch eine Carboxylgruppe substituiert ist, steht.

3. Zusammensetzung nach einem der vorhergehenden Ansprüche, wobei die Dicarbonylverbindung (en) der Formel (I) und/oder Hydrate davon und/oder Salze davon aus Glyoxylsäure und Brenztraubensäure, Salzen davon und Hydraten davon, vorzugsweise aus Glyoxylsäure und den Hydratformen dieser Verbindung, ausgewählt ist bzw. sind.

4. Zusammensetzung nach Anspruch 3, wobei die Glyoxylsäure in ihrer Hydratform vorliegt.

5. Zusammensetzung nach einem der Ansprüche 1 bis 4, umfassend 3 bis 15 Gew.-% einer oder mehrerer Dicarbonylverbindungen der Formel (I) und/oder Hydrate davon und/oder Salze davon, vorzugsweise 5 bis 10 Gew.-%, bezogen auf das Gesamtgewicht der Zusammensetzung.

6. Zusammensetzung nach einem der Ansprüche 1 bis 5, wobei das kationische Polymer bzw. die kationischen Polymere aus den folgenden Polymeren, alleine oder als Mischungen, ausgewählt ist bzw. sind:
(1) Homopolymeren oder Copolymeren, die sich von Acrylsäure- oder Methacrylsäureestern oder -amiden ableiten und mindestens eine der Einheiten der nachstehenden Formeln (I), (II), (III) oder (IV) umfassen: 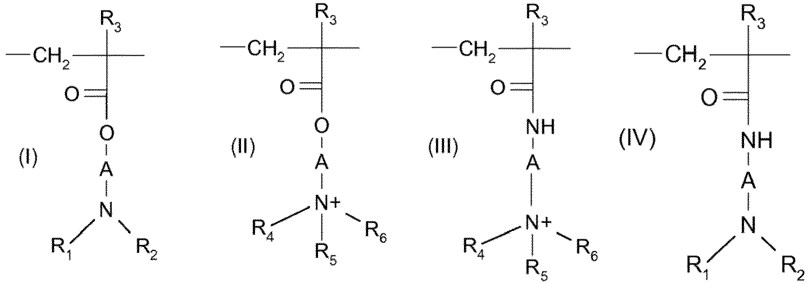 in denen:
R₃, das gleich oder verschieden sein kann, ein Wasserstoffatom oder einen CH₃-Rest bedeutet;
A, das gleich oder verschieden sein kann, für eine lineare oder verzweigte C₁-C₆-Alkylgruppe oder eine Hydroxyalkylgruppe mit 1 bis 4 Kohlenstoffatomen im Alkylteil steht;
R₄, R₅ und R₆, die gleich oder verschieden sein können, für eine Alkylgruppe mit 1 bis 18 Kohlenstoffatomen oder einen Benzylrest stehen;
R₁ und R₂, die gleich oder verschieden sein können, für Wasserstoff oder eine Alkylgruppe mit 1 bis 6 Kohlenstoffatomen stehen;
(2) kationischen Cellulosederivaten;
(3) kationischen Guar-Gummen;
(4) Polymeren, die aus Piperazinyleinheiten und linearen oder verzweigten zweiwertigen Alkyl- oder Hydroxyalkylresten, die gegebenenfalls durch Sauerstoff-, Schwefel- oder Stickstoffatome oder durch aromatische oder heterocyclische Ringe unterbrochen sind, bestehen, sowie den Oxidation- und/oder Quaternisierungsprodukten dieser Polymere;
(5) wasserlöslichen Polyaminoamiden, die gegebenenfalls vernetzt sind;
(6) durch Reaktion eines Polyalkylenpolyamins mit zwei primären Amingruppen und mindestens einer sekundären Amingruppe mit einer Dicarbonsäure erhaltenen Polymeren;
(7) Cyclopolymeren von Alkyldiallylamin oder Dialkyldiallylammonium in Form von Homopolymeren oder Copolymeren, die als Hauptbestandteil der Kette Einheiten umfassen, die der Formel (V) oder (VI) entsprechen: 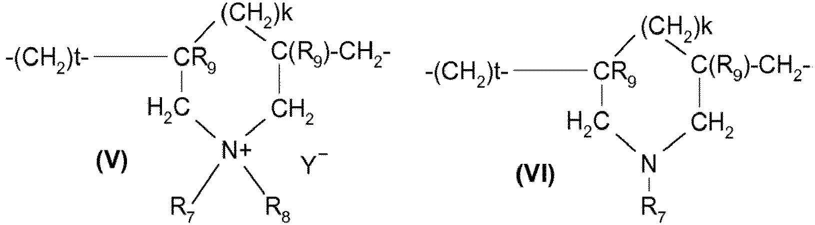 in denen k und t gleich 0 oder 1 sind, die Summe k + t gleich 1 ist; R₉ ein Wasserstoffatom oder einen Methylrest bedeutet; R₇ und R₈ unabhängig voneinander eine C₁-C₈-Alkylgruppe, eine Hydroxyalkylgruppe mit 1 bis 5 Kohlenstoffatomen in der Alkylgruppe oder eine Amidoalkylgruppe mit 1 bis 4 Kohlenstoffatomen im Alkylteil bedeuten; R₇ und R₈ zusammen mit dem Stickstoffatom, an das sie gebunden sind, auch eine heterocyclische Gruppe bedeuten können; Y⁻ für ein organisches oder anorganisches Anion steht;
(8) den diquaternären Ammoniumpolymeren mit Wiederholungseinheiten, die der folgenden Formel entsprechen: 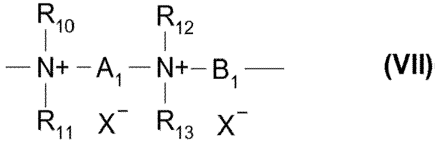 wobei in der Formel (VII):
R₁₀, R₁₁, R₁₂ und R₁₃, die gleich oder verschieden sein können, für lineare, verzweigte oder cyclische, gesättigte, ungesättigte oder aromatische Reste auf C₁-C₂₀-Kohlenwasserstoffbasis, lineare oder verzweigte Hydroxyalkylreste mit 1 bis 4 Kohlenstoffatomen im Alkylteil oder lineare oder verzweigte C₁-C₆-Alkylreste, die durch eine Nitril-, Ester-, Acyl- oder Amidgruppe oder -CO-O-R₁₄-D oder -CO-NH-R₁₄-D substituiert sind, wobei R₁₄ für einen Alkylrest steht und D für eine quaternäre Ammoniumgruppe steht, stehen oder zusammen oder separat mit den Stickstoffatomen, an die sie gebunden sind, Heterocyclen bilden, die gegebenenfalls außer Stickstoff ein zweites Heteroatom enthalten;
A₁ und B₁ für lineare oder verzweigte, gesättigte oder ungesättigte C₂-C₂₀-Reste, die gegebenenfalls durch einen oder mehrere aromatische Ringe, Sauerstoff- oder Schwefelatome oder Gruppen, die mindestens eines dieser Atome tragen, substituiert oder unterbrochen sind, stehen;
X⁻ ein organisches oder anorganisches Anion bedeutet;
A₁, R₁₀ und R₁₂ mit den beiden Stickstoffatomen, an die sie gebunden sind, einen Piperazinring bilden können; außerdem B₁ dann, wenn A₁ einen linearen oder verzweigten, gesättigten oder ungesättigten Alkylen- oder Hydroxyalkylenrest bedeutet, auch eine -(CH₂)ₙCO-D-OC(CH₂)ₙ-Gruppe bedeuten kann, in der n zwischen 1 und 100 liegt und D einen Glykol-, bissekundären Diamin-, bisprimären Diamin- oder Ureylenrest bedeutet;
(9) polyquaternären Ammoniumpolymeren, die aus Wiederholungseinheiten der Formel (IX) bestehen: 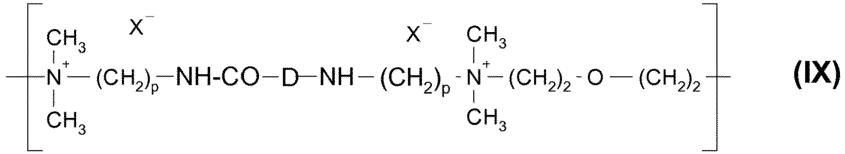 in der p eine ganze Zahl im Bereich von 1 bis 6 bedeutet, D null sein kann oder für eine -(CH₂)ᵣ-CO-Gruppe, in der r eine Zahl mit einem Wert von 4 oder 7 bedeutet, stehen kann und X⁻ für ein organisches oder anorganisches Anion steht;
(10) quaternären Polymeren von Vinylpyrrolidon und Vinylimidazol;
(11) Polyaminen.

7. Zusammensetzung nach Anspruch 6, **dadurch gekennzeichnet, dass** das kationische Polymer bzw. die kationischen Polymere aus den Polymeren (1), (2), (3), (7) und (8), vorzugsweise aus den Polymeren (1), (2), (7) und (8) und noch besser aus den Polymeren (1), (7) und (8), allein oder als Mischungen, ausgewählt ist bzw. sind.

8. Zusammensetzung nach einem der Ansprüche 1 bis 5, wobei das amphotere Polymer bzw. die amphoteren Polymere aus den folgenden Verbindungen ausgewählt ist bzw. sind:
(1) Polymeren, die als Monomere mindestens ein Monomer, das sich von einer Vinylverbindung mit einer Carboxylgruppe, vorzugsweise Acrylsäure, Methacrylsäure, Maleinsäure, α-Chloracrylsäure, ableitet, und mindestens ein basisches Monomer, das sich von einer substituierten Vinylverbindung mit mindestens einem basischen Atom ableitet und ausgewählt ist aus:
a) Dialkylaminoalkylmethacrylaten, Dialkylaminoalkylacrylaten, Dialkylaminoalkylmethacrylamiden und Dialkylaminoalkylacrylamiden,
b) Trialkylaminoalkylmethacrylatsalzen, Trialkylaminoalkylacrylatsalzen Trialkylaminoalkylmethacrylamidsalzen und Trialkylaminoalkylacrylamidsalzen,
umfassen,
(2) Polymeren, die Einheiten umfassen, die sich von:
a) mindestens einem Monomer, das aus Acrylamiden oder Methacrylamiden, die am Stickstoff mit einem Alkylrest, der vorzugsweise 2 bis 12 Kohlenstoffatome enthält, substituiert sind, ausgewählt ist,
b) mindestens einem sauren Comonomer mit einer oder mehreren reaktiven Carboxylgruppen, die vorzugsweise aus Acrylsäure, Methacrylsäure, Crotonsäure, Itaconsäure, Maleinsäure und Fumarsäure und auch Alkylmonoestern, die 1 bis 4 Kohlenstoffatome enthalten, von Maleinsäure oder Fumarsäure oder deren Anhydriden ausgewählt sind,
c) mindestens einem basischen Comonomer wie Estern mit primären, sekundären, tertiären und quaternären Aminsubstituenten von Acrylsäure und Methacrylsäure und dem Produkt der Quaternisierung von Dimethylaminoethylmethacrylat mit Dimethyl- oder Diethylsulfat
ableiten,
(3) Copolymeren, die als Monomere mindestens ein Monomer, das sich von einer Vinylverbindung mit einer Carboxylgruppe, wie spezieller Acrylsäure, Methacrylsäure, Maleinsäure, α-Chloracrylsäure, ableitet, und mindestens ein Monomer vom Diallyldialkylammoniumsalz-Typ, wobei die Alkylgruppen 1 bis 6 Kohlenstoffatome enthalten, umfassen;
(4) vernetzten und alkylierten Polyaminoamiden, die sich ganz oder teilweise von Polyaminoamiden der allgemeinen Formel:
-⁅CO-R₁₀-CO-Z⁆- **(I')**
ableiten, in der R₁₀ für einen zweiwertigen Rest, der sich von einer gesättigten Dicarbonsäure, einer aliphatischen Mono- oder Dicarbonsäure mit einer ethylenischen Doppelbindung, einem Ester dieser Säuren mit einem niederen Alkanol mit 1 bis 6 Kohlenstoffatomen oder einem Rest, der sich aus der Addition einer dieser Säuren an ein bis(primäres) oder bis(sekundäres) Amin ergibt, ableitet, steht und Z einen Rest, der sich von einem bis(primären), mono- oder bis(sekundären) Polyalkylenpolyamin ableitet, bedeutet und vorzugsweise für:
a) in Anteilen von 60 bis 100 Mol-%, den Rest: 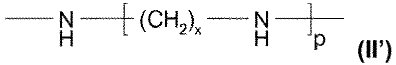
wobei x = 2 und p = 2 oder 3 oder auch x = 3 und p = 2,
wobei sich dieser Rest von Diethylentriamin, Triethylentetramin oder Dipropylentriamin ableitet;
b) in Anteilen von 0 bis 40 Mol-%, den obigen Rest (II'), in dem x = 2 und p = 1 und der sich von Ethylendiamin ableitet, oder den Rest, der sich von Piperazin ableitet: 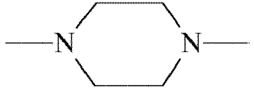
c) in Anteilen von 0 bis 20 Mol-%, den Rest -NH-(CH₂)₆-NH-, der sich von Hexamethylendiamin ableitet,
steht, wobei diese Polyaminoamine durch Zugabe eines difunktionellen Vernetzungsmittels, das aus Epihalogenhydrinen, Diepoxiden, Dianhydriden und zweifach ungesättigten Derivaten ausgewählt ist, mittels 0,025 bis 0,35 mol Vernetzungsmittel pro Amingruppe des Polyaminoamids vernetzt sind und durch Einwirkung von Acrylsäure, Chloressigsäure oder einem Alkansulton oder Salzen davon alkyliert sind,
(5) Polymeren, die zwitterionische Einheiten der Formel: 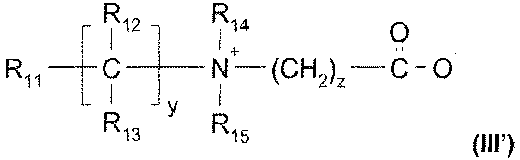 umfassen, in der R₁₁ eine polymerisierbare ungesättigte Gruppe, wie eine Acrylat-, Methacrylat-, Acrylamid- oder Methacrylamidgruppe, bedeutet, y und z für eine ganze Zahl von 1 bis 3 stehen, R₁₂ und R₁₃ für ein Wasserstoffatom, Methyl, Ethyl oder Propyl stehen und R₁₄ und R₁₅ für ein Wasserstoffatom oder einen solchen Alkylrest stehen, dass die Summe der Kohlenstoffatome in R₁₄ und R₁₅ nicht mehr als 10 beträgt,
(6) Polymeren, die sich von Chitosan ableiten, umfassend Monomereinheiten, die den folgenden Formeln entsprechen: 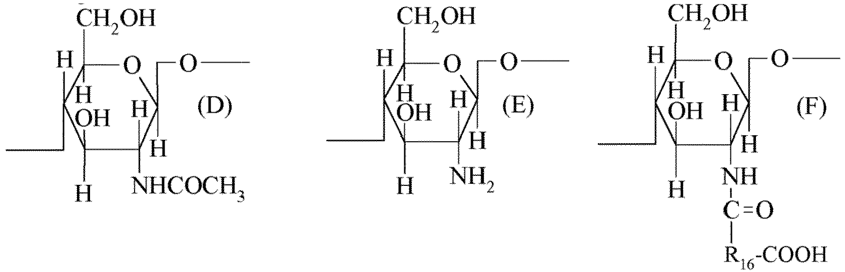 wobei die Einheit D in Anteilen zwischen 0 und 30 % vorliegt, die Einheit E in Anteilen zwischen 5 und 50 % vorliegt und die Einheit F in Anteilen zwischen 30 und 90 % vorliegt, wobei es sich versteht, dass in dieser Einheit F R₁₆ für einen Rest der Formel: 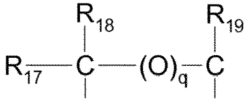
steht, in der dann, wenn q = 0, R₁₇, R₁₈ und R₁₉, die gleich oder verschieden sein können, jeweils für ein Wasserstoffatom, einen Methyl-, Hydroxyl-, Acetoxy- oder Aminorest, einen Monoalkylaminrest oder einen Dialkylaminrest, die gegebenenfalls durch ein oder mehrere Stickstoffatome unterbrochen und/oder gegebenenfalls durch eine oder mehrere Amin-, Hydroxyl-, Carboxyl-, Alkylthio- oder Sulfonsäuregruppen substituiert sind, oder einen Alkylthiorest, in dem die Alkylgruppe einen Aminorest trägt, stehen, wobei in diesem Fall mindestens einer der Reste R₁₇, R₁₈ und R₁₉ für ein Wasserstoffatom steht;
oder dann, wenn q = 1, R₁₇, R₁₈ und R₁₉ jeweils für ein Wasserstoffatom stehen, sowie den durch diese Verbindungen mit Basen oder Säuren gebildeten Salzen;
(7) Polymeren, die sich aus der N-Carboxyalkylierung von Chitosan ergeben;
(8) Polymeren mit Einheiten, die der allgemeinen Formel (IV') entsprechen: 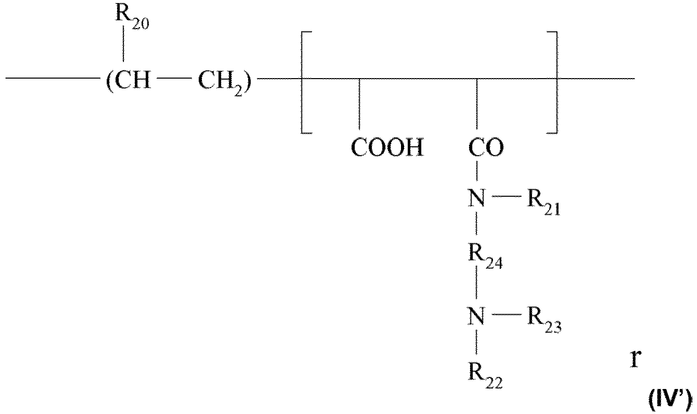
in der R₂₀ für ein Wasserstoffatom, eine CH₃O-, CH₃CH₂O- oder Phenylgruppe steht, R₂₁ ein Wasserstoffatom oder einen Niederalkylrest wie Methyl oder Ethyl bedeutet, R₂₂ ein Wasserstoffatom oder einen Niederalkylrest wie Methyl oder Ethyl bedeutet, R₂₃ einen Niederalkylrest wie Methyl oder Ethyl oder einen Rest, der der Formel -R₂₄-N(R₂₂)₂ entspricht, bedeutet, wobei R₂₄ für eine -CH₂-CH₂-, -CH₂-CH₂-CH₂- oder -CH₂-CH(CH₃)-Gruppe steht, wobei R₂₂ die oben angegebenen Bedeutungen besitzt,
sowie die höheren Homologen dieser Reste, die bis zu 6 Kohlenstoffatome enthalten;
(9) amphoteren Polymeren des -D-X-D-X-Typs, ausgewählt aus:
a) durch Umsetzung von Chloressigsäure oder Natriumchloracetat mit Verbindungen, die mindestens eine Einheit der Formel:
-D-X-D-X-D- (**V'**)
umfassen, erhaltenen Polymeren, wobei D einen Rest 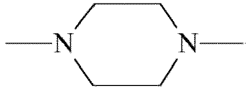 bedeutet und X das Symbol E oder E' bedeutet, E oder E', die gleich oder verschieden sein können, einen zweiwertigen Rest bedeuten, bei dem es sich um einen Alkylenrest mit gerader oder verzweigter Kette und bis zu 7 Kohlenstoffatomen in der Hauptkette handelt, der unsubstituiert oder durch Hydroxylgruppen substituiert ist und neben den Sauerstoff-, Stickstoff- und Schwefelatomen 1 bis 3 aromatische und/oder heterocyclische Ringe umfassen kann; wobei die Sauerstoff-, Stickstoff- und Schwefelatome in Form von Ether-, Thioether-, Sulfoxid-, Sulfon-, Sulfonium-, Alkylamin- oder Alkenylamingruppen, Hydroxyl-, Benzylamin-, Aminoxid-, quaternären Ammonium-, Amid-, Imid-, Alkohol-, Ester- und/oder Urethangruppen vorliegen,
b) Polymeren der Formel:
-D-X-D-X- (**VI'**)
wobei D einen Rest 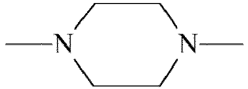 bedeutet und X das Symbol E oder E' und mindestens einmal E' bedeutet; wobei E die oben angegebene Bedeutung besitzt und E' für einen zweiwertigen Rest steht, bei dem es sich um einen Alkylenrest mit gerader oder verzweigter Kette und bis zu 7 Kohlenstoffatomen in der Hauptkette handelt, der unsubstituiert oder durch einen oder mehrere Hydroxylreste substituiert ist und ein oder mehrere Stickstoffatome umfasst, wobei das Stickstoffatom durch eine Alkylkette substituiert ist, die gegebenenfalls durch ein Sauerstoffatom unterbrochen ist und zwingend eine oder mehrere Carboxylfunktionen oder eine oder mehrere Hydroxylfunktionen umfasst und durch Umsetzung mit Chloressigsäure oder Natriumchloracetat betainisiert ist,
(10) (C₁-C₅)Alkylvinylether/Maleinsäureanhydrid-Copolymeren, die durch Teilamidierung mit einem N,N-Dialkylaminoalkylamin oder durch Teilveresterung mit einem N,N-Dialkanolamin teilweise modifiziert sind;
und Mischungen davon.

9. Zusammensetzung nach einem der vorhergehenden Ansprüche, **dadurch gekennzeichnet, dass** das amphotere Polymer bzw. die amphoteren Polymere aus Copolymeren, die als Monomere mindestens ein Monomer, das sich von einer Vinylverbindung mit einer Carboxylgruppe, wie Acrylsäure, Methacrylsäure, Maleinsäure oder α-Chloracrylsäure, ableitet, und mindestens ein Monomer vom Diallyldialkylammoniumsalz-Typ, wobei die Alkylgruppen 1 bis 6 Kohlenstoffatome enthalten, umfassen, speziell aus Copolymeren, die als Monomere Dimethyldiallylammoniumchlorid und Acrylsäure, gegebenenfalls in Kombination mit Acrylamid, umfassen, ausgewählt ist bzw. sind.

10. Zusammensetzung nach einem der vorhergehenden Ansprüche, wobei der Gehalt der kationischen Polymere im Bereich von 0,01 bis 20 Gew.-% und vorzugsweise von 0,1 bis 10 Gew.-%, bezogen auf das Gesamtgewicht der Zusammensetzung, liegt.

11. Zusammensetzung nach einem der vorhergehenden Ansprüche, wobei das Gewichtsverhältnis von Dicarbonylverbindungen gemäß Formel (I) und/oder Hydratform davon und/oder Salzen davon zu kationischem Polymer bzw. kationischen Polymeren oder kationischem und amphoterem Polymer bzw. kationischen und amphoteren Polymeren im Bereich von 1 bis 50 und noch besser von 2 bis 20 liegt.

12. Zusammensetzung nach einem der vorhergehenden Ansprüche, **dadurch gekennzeichnet, dass** sie wässrig ist und Wasser in einer Konzentration, die vorzugsweise im Bereich von 5 bis 98 Gew.-%, noch besser von 5 bis 90 Gew.-% und sogar noch besser von 10 bis 90 Gew.-%, bezogen auf das Gesamtgewicht der Zusammensetzung, liegt, umfasst.

13. Zusammensetzung nach einem der vorhergehenden Ansprüche, **dadurch gekennzeichnet, dass** sie einen pH-Wert von weniger als 4 aufweist, wobei der pH-Wert vorzugsweise im Bereich von 1 bis 3, noch besser von 1,5 bis 3 und sogar noch besser von 1,7 bis 3 liegt.

14. Zusammensetzung nach einem der vorhergehenden Ansprüche, **dadurch gekennzeichnet, dass** sie sich aus dem Mischen mehrerer Zusammensetzungen ergibt.

15. Verfahren zum Glätten von Keratinfasern, insbesondere des Haars, umfassend das Aufbringen der Zusammensetzung nach einem der vorhergehenden Ansprüche auf das Haar, vorzugsweise gefolgt von einer Kontaktzeit von 10 bis 60 Minuten, und dann einem Glättungsschritt unter Verwendung eines Glätteisens bei einer Temperatur von mindestens 150 °C, vorzugsweise im Bereich von 150 bis 250 °C.

16. Verwendung der Zusammensetzung nach einem der Ansprüche 1 bis 14 zum Glätten/Entkräuseln von Keratinfasern, insbesondere dem Haar.

## Revendications

1. Composition cosmétique comprenant au moins un polymère cationique ou au moins un polymère cationique et un polymère amphotère et au moins 3 % en poids par rapport au poids total de la composition d'un ou plusieurs composés dicarbonyle correspondant à la formule (I) ci-dessous et/ou d'hydrates de ceux-ci et/ou de sels de ceux-ci : 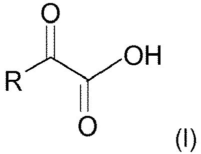 dans laquelle formule (I) :
R représente un atome ou groupe choisi entre i) l'atome d'hydrogène, ii) le groupe carboxyle -C(O)OH, iii) un groupe alkyle en C₁-C₆ linéaire ou ramifié qui est éventuellement substitué, de préférence par au moins un radical hydroxyle -OH, radical carboxyle -C(O)-OH ou radical d'halogène tel que Br ; iv) un groupe phényle éventuellement substitué, v) un groupe benzyle éventuellement substitué, iv) et v) étant de préférence éventuellement substitués par au moins un radical -OH ou -C(O)OH ; vi) un radical indolyle et vii) un radical imidazolylméthyle et les tautomères de celui-ci tels que 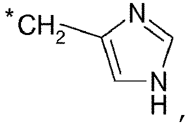 * représentant la partie liée au reste de la molécule.

2. Composition selon la revendication 1, dans laquelle R représente i) un atome d'hydrogène ou ii) un groupe alkyle en C₁-C₆ linéaire ou ramifié éventuellement substitué par un groupe carboxyle.

3. Composition selon l'une ou l'autre des revendications précédentes, dans laquelle le ou les composés dicarbonyle correspondant à la formule (I) et/ou les hydrates de ceux-ci et/ou les sels de ceux-ci sont choisis parmi l'acide glyoxylique et l'acide pyruvique, les sels de ceux-ci et les hydrates de ceux-ci, de préférence parmi l'acide glyoxylique et les formes hydratées de ce composé.

4. Composition selon la revendication 3, dans laquelle l'acide glyoxylique est sous sa forme hydratée.

5. Composition selon l'une quelconque des revendications 1 à 4, comprenant de 3 % à 15 % en poids d'un ou plusieurs composés dicarbonyle correspondant à la formule (I) et/ou d'hydrates de ceux-ci et/ou de sels de ceux-ci, de préférence de 5 % à 10 % en poids par rapport au poids total de la composition.

6. Composition selon l'une quelconque des revendications 1 à 5, dans laquelle le ou les polymères cationiques sont choisis parmi les polymères suivants, seuls ou sous forme de mélanges :
(1) les homopolymères ou copolymères dérivés d'esters ou amides acryliques ou méthacryliques et comprenant au moins l'un des motifs de formule (I), (II), (III) ou (IV) ci-dessous : 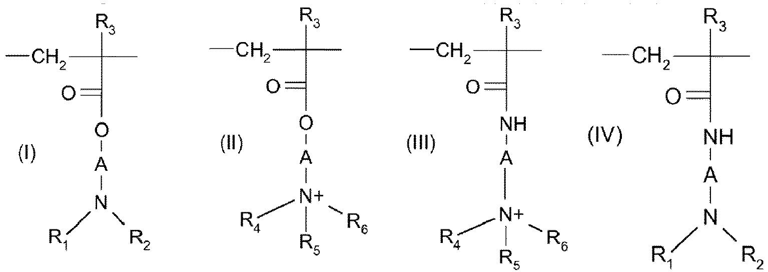 dans lesquels :
R₃, qui peut être identique ou différent, désigne un atome d'hydrogène ou un radical CH₃ ;
A, qui peut être identique ou différent, représente un groupe alkyle en C₁-C₆ linéaire ou ramifié ou un groupe hydroxyalkyle, dont le groupe alkyle est en C₁-C₄ ;
R₄, R₅ et R₆, qui peuvent être identiques ou différents, représentent un groupe alkyle en C₁-C₁₈ ou un radical benzyle ;
R₁ et R₂, qui peuvent être identiques ou différents, représentent l'atome d'hydrogène ou un groupe alkyle en C₁-C₆ ;
(2) les dérivés cationiques de cellulose ;
(3) les gommes de guar cationiques ;
(4) les polymères constitués de motifs pipérazinyle et de radicaux alkyle ou hydroxyalkyle divalents linéaires ou ramifiés, éventuellement interrompus par des atomes d'oxygène, de soufre ou d'azote ou par des cycles aromatiques ou hétérocycliques, et également les produits d'oxydation et/ou de quaternisation de ces polymères ;
(5) les polyaminoamides hydrosolubles qui sont éventuellement réticulés ;
(6) les polymères obtenus par réaction d'une polyalkylènepolyamine contenant deux groupes amine primaire et au moins un groupe amine secondaire avec un acide dicarboxylique ;
(7) les cyclopolymères d'alkyldiallyamine ou de dialkyldiallylammonium, sous la forme d'homopolymères ou de copolymères contenant, en tant que constituants principaux de la chaîne, des motifs correspondant à la formule (V) ou (VI) : 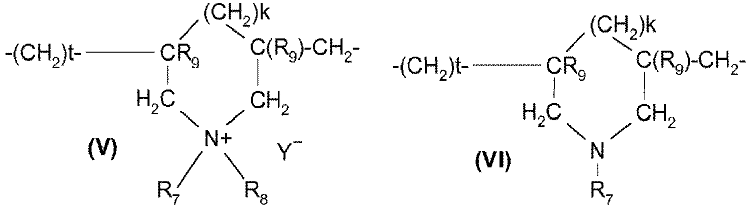 dans lesquelles formules
k et t sont égaux à 0 ou 1, la somme k + t étant égale à 1 ;
R₉ désigne un atome d'hydrogène ou un radical méthyle ;
R₇ et R₈, indépendamment l'un de l'autre, désignent chacun un groupe alkyle en C₁-C₈, un groupe hydroxyalkyle dans lequel le groupe alkyle est en C₁-C₅, un groupe amidoalkyle dans lequel le groupe alkyle est en C₁-C₄ ; R₇ et R₈ peuvent également désigner, conjointement avec l'atome d'azote auquel ils sont liés, un groupe hétérocyclique ;
Y⁻ est un anion organique ou minéral ;
(8) les polymères de diammonium quaternaire contenant des motifs répétés correspondant à la formule : 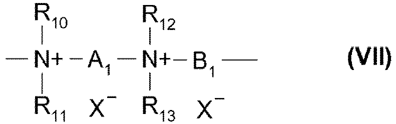 dans laquelle formule (VII) :
R₁₀, R₁₁, R₁₂ et R₁₃, qui peuvent être identiques ou différents, représentent des radicaux à base d'hydrocarbures en C₁-C₂₀ linéaires, ramifiés ou cycliques, saturés, insaturés ou aromatiques, des radicaux hydroxyalkyle linéaires ou ramifiés dans lesquels la partie alkyle est en C₁-C₄, des radicaux alkyle en C₁-C₆ linéaires ou ramifiés, substitués par un groupe nitrile, ester, acyle ou amide ou -CO-O-R₁₄-D ou -CO-NH-R₁₄-D, R₁₄ représentant un radical alkyle et D un groupe ammonium quaternaire, ou forment, ensemble ou séparément, avec les atomes d'azote auxquels ils sont liés, des hétérocycles contenant éventuellement un second hétéroatome autre que l'atome d'azote ;
A₁ et B₁ représentent des radicaux en C₂-C₂₀ linéaires ou ramifiés, saturés ou insaturés, éventuellement substitués ou interrompus par un ou plusieurs cycles aromatiques, atomes d'oxygène ou de soufre ou groupes renfermant au moins l'un de ces atomes ;
X⁻ désigne un anion organique ou minéral ;
A₁, R₁₀ et R₁₂ peuvent former, avec les deux atomes d'azote auxquels ils sont liés, un cycle pipérazine ;
de plus, si A₁ désigne un radical alkylène ou hydroxyalkylène linéaire ou ramifié, saturé ou insaturé, B₁ peut également désigner un groupe -(CH₂)ₙCO-D-OC(CH₂)ₙ- dans lequel n est compris entre 1 et 100 et D désigne un résidu de glycol, de diamine deux fois secondaire, de diamine deux fois primaire ou d'uréylène ;
(9) les polymères de polyammonium quaternaire constitués de motifs répétés de formule (IX) : 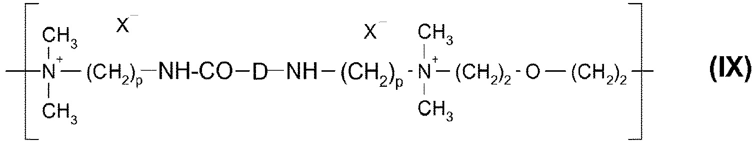 dans lesquels
p désigne un nombre entier allant de 1 à 6,
D peut être absent ou peut représenter un groupe -(CH₂)ᵣ-CO- dans lequel r désigne un nombre égal à 4 ou 7 et
X⁻ est un anion organique ou minéral ;
(10) les polymères quaternaires de vinylpyrrolidone et de vinylimidazole ;
(11) les polyamines.

7. Composition selon la revendication 6, **caractérisée en ce que** le ou les polymères cationiques sont choisis parmi les polymères (1), (2), (3), (7) et (8), de préférence parmi les polymères (1), (2), (7) et (8) et mieux encore parmi les polymères (1), (7) et (8), seuls ou sous forme de mélanges.

8. Composition selon l'une quelconque des revendications 1 à 5, dans laquelle le ou les polymères amphotères sont choisis parmi les composés suivants :
(1) les polymères comprenant, en tant que monomères, au moins un monomère dérivé d'un composé vinylique portant un groupe carboxylique, de préférence d'acide acrylique, d'acide méthacrylique, d'acide maléique, d'acide α-chloroacrylique, et au moins un monomère basique dérivé d'un composé vinylique substitué contenant au moins un atome basique choisi parmi :
a) les méthacrylates de dialkylaminoalkyle, les acrylates de dialkylaminoalkyle, les dialkylaminoalkylméthacrylamides et les dialkylaminoacrylamides ;
b) les sels de méthacrylates de trialkylaminoalkyle, les sels d'acrylates de trialkylaminoalkyle, les sels de trialkylaminoalkylméthacrylamides et les sels de trialkylaminoalkylacrylamides ;
(2) les polymères comprenant des motifs dérivés de :
a) au moins un monomère choisi parmi les acrylamides ou méthacrylamides substitués sur l'atome d'azote par un radical alkyle de préférence contenant de 2 à 12 atomes de carbone ;
b) au moins un comonomère acide contenant un ou plusieurs groupes carboxyliques réactifs, de préférence choisi parmi l'acide acrylique, l'acide méthacrylique, l'acide crotonique, l'acide itaconique, l'acide maléique et l'acide fumarique et également parmi les monoesters alkyliques, contenant 1 à 4 atomes de carbone, des acides ou anhydrides maléique ou fumarique ;
c) au moins un comonomère basique tel que des esters contenant des substituants amine primaire, secondaire, tertiaire et quaternaire des acides acrylique et méthacrylique et le produit de quaternisation de méthacrylate de diméthylaminoéthyle avec du sulfate de diméthyle ou de diéthyle ;
(3) les copolymères comprenant, en tant que monomères, au moins un monomère dérivé d'un composé vinylique portant un groupe carboxylique, tel que, plus particulièrement, l'acide acrylique, l'acide méthacrylique, l'acide maléique, l'acide α-chloroacrylique, et au moins un monomère de type sel de dialkyldiallylammonium, les groupes alkyle contenant de 1 à 6 atomes de carbone ;
(4) les polyaminoamides réticulés et alkylés dérivés en partie ou en totalité de polyaminoamides de formule générale :
-⁅CO-R₁₀-CO-Z⁆- **(I')**
dans lesquels
R¹⁰ représente un radical divalent dérivé d'un acide dicarboxylique saturé, d'un acide aliphatique monocarboxylique ou dicarboxylique contenant une double liaison éthylénique, d'un ester d'un alcanol inférieur contenant 1 à 6 atomes de carbone de ces acides ou un radical dérivé de l'addition de l'un quelconque desdits acides sur une amine deux fois primaire ou deux fois secondaire et
Z désigne un radical provenant d'une polyalkylènepolyamine deux fois primaire, une fois secondaire ou deux fois secondaire et de préférence représente :
a) en proportions de 60 à 100 % en mole, le radical 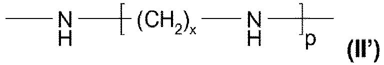
où x = 2 et p = 2 ou 3, ou en variante x = 3 et p = 2,
ce radical étant dérivé de diéthylènetriamine, de triéthylènetétraamine ou de dipropylènetriamine ;
b) en proportions de 0 à 40 % en mole, le radical (II') ci-dessus dans lequel x = 2 et p = 1 et qui est dérivé d'éthylènediamine ou le radical dérivé de pipérazine : 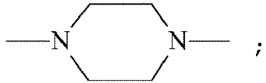
c) en proportions de 0 à 20 % en mole, le radical -NH(CH₂)₆-NH- qui est dérivé d'hexaméthylènediamine,
ces polyaminoamines étant réticulées par ajout d'un agent de réticulation difonctionnel choisi parmi les épihalogénhydrines, les diépoxydes, les dianhydrides et les dérivés à deux insaturations, à l'aide de 0,025 à 0,35 mol d'agent de réticulation par groupe amine du polyaminoamide et alkylées par l'action d'acide acrylique, d'acide chloroacétique ou d'une alcanesultone, ou de sels de ceux-ci ;
(5) les polymères comprenant des motifs zwitterioniques de formule : 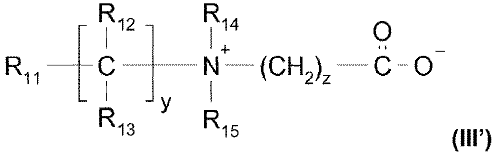 dans lesquels
R₁₁ désigne un groupe insaturé polymérisable tel qu'un groupe acrylate, méthacrylate, acrylamide ou méthacrylamide,
y et z représentent un nombre entier de 1 à 3,
R₁₂ et R₁₃ représentent un atome d'hydrogène ou un groupe méthyle, éthyle ou propyle,
R₁₄ et R₁₅ représentent un atome d'hydrogène ou un radical alkyle de façon telle que la somme des nombres d'atomes de carbone présents dans R₁₄ et R₁₅ ne dépasse pas 10 ;
(6) les polymères dérivés de chitosane comprenant des motifs monomères correspondant aux formules suivantes : 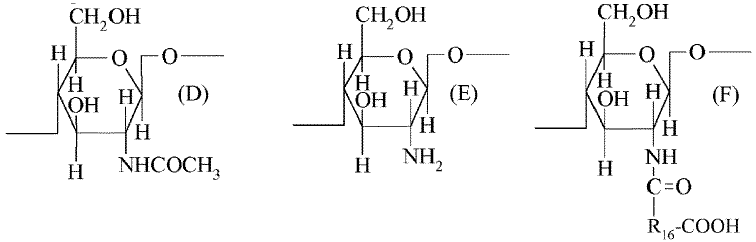 le motif D étant présent en proportions comprises entre 0 et 30 %, le motif E en proportions comprises entre 5 % et 50 % et le motif F en proportions comprises entre 30 % et 90 %, étant entendu que, dans ce motif F, R₁₆ représente un radical de formule : 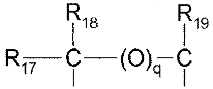 dans lequel,
si q = 0, R₁₇, R₁₈ et R₁₉, qui peuvent être identiques ou différents, représentent chacun un atome d'hydrogène, un résidu méthyle, hydroxyle, acétoxy ou amino, un résidu de monoalkylamine ou un résidu de dialkylamine qui sont éventuellement interrompus par un ou plusieurs atomes d'azote et/ou éventuellement substitués par un ou plusieurs groupes amine, hydroxyle, carboxyle, alkylthio ou sulfoniques, ou un résidu alkylthio dans lequel le groupe alkyle porte un résidu amino, au moins l'un des radicaux R₁₇, R₁₈ et R₁₉ étant, dans ce cas, un atome d'hydrogène ;
ou, si q = 1, R₁₇, R₁₈ et R₁₉ représentent chacun un atome d'hydrogène,
et également les sels formés par ces composés avec des bases ou des acides ;
(7) les polymères dérivés de la N-carboxyalkylation de chitosane ;
(8) les polymères contenant des motifs correspondant à la formule générale (IV') : 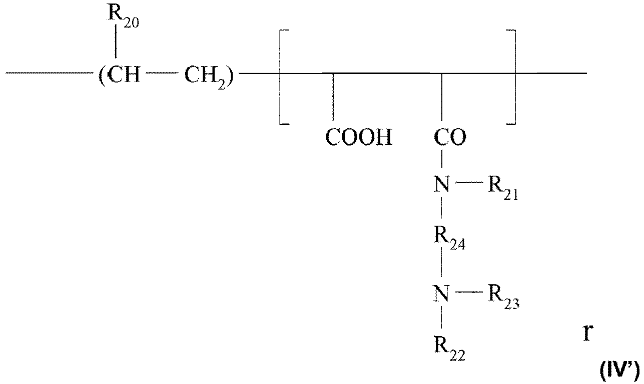 dans lesquels
R₂₀ représente un atome d'hydrogène, un radical CH₃O, CH₃CH₂O ou phényle,
R₂₁ désigne l'atome d'hydrogène ou un radical alkyle inférieur tel qu'un radical méthyle ou éthyle,
R₂₂ désigne l'atome d'hydrogène ou un radical alkyle inférieur tel qu'un radical méthyle ou éthyle,
R₂₃ désigne un radical alkyle inférieur tel qu'un radical méthyle ou éthyle ou un radical correspondant à la formule : -R₂₄-N(R₂₂)₂, R₂₄ représentant un groupe -CH₂-CH₂-, -CH₂-CH₂-CH₂- ou -CH₂-CH(CH₃)-, R₂₂ ayant les significations mentionnées ci-dessus,
et également les homologues supérieurs de ces radicaux, contenant jusqu'à 6 atomes de carbone ;
(9) les polymères amphotères du type -D-X-D-X choisis parmi :
a) les polymères obtenus par l'action d'acide chloroacétique ou de chloroacétate de sodium sur des composés comprenant au moins un motif de formule :
-D-X-D-X-D- **(V')**
où
D désigne un radical 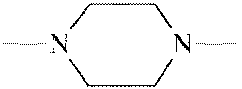 et
X désigne le symbole E ou E',
E ou E', qui peuvent être identiques ou différents, désignent chacun un radical divalent qui est un radical alkylène à chaîne droite ou ramifiée contenant jusqu'à 7 atomes de carbone dans la chaîne principale, qui est non substitué ou substitué par des groupes hydroxyle et qui peut comprendre, en plus d'atomes d'oxygène, d'azote et de soufre, 1 à 3 cycles aromatiques et/ou hétérocycliques ; les atomes d'oxygène, d'azote et de soufre étant présents sous la forme de groupes éther, thioéther, sulfoxyde, sulfone, sulfonium, alkylamine ou alkylèneamine, hydroxyle, benzylamine, oxyde d'amine, ammonium quaternaire, amide, imide, alcool, ester et/ou uréthane ;
b) les polymères de formule :
-D-X-D-X- **(VI')**
où
D désigne un radical 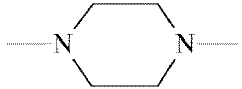 et
X désigne le symbole E ou E' et au moins une fois E' ; E ayant la signification donnée ci-dessus et E' étant un radical divalent qui est un radical alkylène à chaîne droite ou ramifiée contenant jusqu'à 7 atomes de carbone dans la chaîne principale, qui est non substitué ou substitué par un ou plusieurs radicaux hydroxyle et contenant un ou plusieurs atomes d'azote, l'atome d'azote étant substitué par une chaîne alkyle qui est éventuellement interrompue par un atome d'oxygène et comprenant obligatoirement une ou plusieurs fonctions carboxyle ou une ou plusieurs fonctions hydroxyle et bétaïnisé par réaction avec de l'acide chloroacétique ou du chloroacétate de sodium ;
(10) les copolymères d'éther d'alkyle en C₁-C₅ et de vinyle/anhydride maléique en partie modifiés par semi-amidation avec une *N,N*-dialkylaminoalkylamine ou par semi-estérification avec une *N,N*-dialcanolamine ;
et les mélanges de ceux-ci.

9. Composition selon l'une quelconque des revendications précédentes, **caractérisée en ce que** le ou les polymères amphotères sont choisis parmi les copolymères comprenant en tant que monomères au moins un monomère dérivé d'un composé vinylique portant un groupe carboxylique, tel que l'acide acrylique, l'acide méthacrylique, l'acide maléique ou l'acide α-chloroacrylique, et au moins un monomère de type sel de dialkyldiallylammonium, les groupes alkyle contenant de 1 à 6 atomes de carbone, plus particulièrement parmi les copolymères comprenant en tant que monomères le chlorure de diallyldiméthylammonium et l'acide acrylique, éventuellement combinés avec l'acrylamide.

10. Composition selon l'une quelconque des revendications précédentes, dans laquelle la teneur de polymères cationiques va de 0,01 % à 20 % en poids et de préférence de 0,1 % à 10 % en poids par rapport au poids total de la composition.

11. Composition selon l'une quelconque des revendications précédentes, dans laquelle le rapport pondéral de composés dicarbonyle correspondant à la formule (I) et/ou de forme hydratée de ceux-ci et/ou de sels de ceux-ci aux polymères cationiques ou aux polymères cationiques et amphotères va de 1 à 50 et mieux encore de 2 à 20.

12. Composition selon l'une quelconque des revendications précédentes, **caractérisée en ce qu'**elle est aqueuse et comprend de l'eau en une concentration de préférence allant de 5 % à 98 %, mieux encore de 5 % à 90 % même mieux encore de 10 % à 90 % en poids par rapport au poids total de la composition.

13. Composition selon l'une quelconque des revendications précédentes, **caractérisée en ce qu'**elle a un pH inférieur à 4, le pH de préférence allant de 1 à 3, mieux encore de 1,5 à 3 et même mieux encore de 1,7 à 3.

14. Composition selon l'une quelconque des revendications précédentes, **caractérisée en ce qu'**elle résulte du mélange de plusieurs compositions.

15. Procédé pour le lissage de fibres de kératine, en particulier de cheveux, qui comprend l'application aux cheveux de la composition selon l'une quelconque des revendications précédentes, de préférence suivie d'un temps de contact allant de 10 à 60 minutes et ensuite une étape de lissage à l'aide d'un lisseur à une température d'au moins 150 °C, de préférence allant de 150 à 250 °C.

16. Utilisation de la composition selon l'une quelconque des revendications 1 à 14, pour le lissage/défrisage de fibres de kératine, en particulier de cheveux.
